(19) Europäisches Patentamt
European Patent Office
Office européen des brevets

(11) Publication number: **0 179 660**
**A2**

# (12) EUROPEAN PATENT APPLICATION

(21) Application number: 85307674.3

(22) Date of filing: 24.10.85

(51) Int. Cl.[4]: A 61 K 9/50

(30) Priority: 26.10.84 US 665273

(43) Date of publication of application: 30.04.86 Bulletin 86/18

(84) Designated Contracting States: AT BE CH DE FR GB IT LI LU NL SE

(71) Applicant: ELI LILLY AND COMPANY
Lilly Corporate Center
Indianapolis Indiana 46285(US)

(72) Inventor: Pidgeon, Charles
590 West Linden Street
Zionsville Indiana 46077(US)

(74) Representative: Hudson, Christopher Mark et al,
Erl Wood Manor
Windlesham Surrey GU20 6PH(GB)

## (54) Multilayered vesicles prepared by reverse-phase evaporation.

(57) The present invention provides a method of preparing multilayered vesicles under conditions which allow predicting the number of bilayers and amount of drug entrapment in the vesicles. Novel liposome suspensions prepared by the present process are also provided as another embodiment of the present invention.

EP 0 179 660 A2

Croydon Printing Company Ltd.

# MULTILAYERED VESICLES PREPARED BY REVERSE-PHASE EVAPORATION

Extensive research has continued for a number of years directed at formulating drugs in order to achieve improved therapy to better control pharmacological activity of drugs. In most drug delivery systems the process of delivering therapeutic agents to their sites of action within the body proceeds in a rather inefficient manner. The drug may be metabolized to an inactive compound or may be the cause of serious side effects due to the compound's random route within the body. Therefore, an improved drug delivery system is one capable of efficiently delivering the minimum amount of drug necessary to produce the desired therapeutic response while minimizing unwanted side effects. These drug delivery systems have the ability to alter the disposition of the drug by controlled release throughout the body, or deliver the drug to the target site. Consequently, this type of drug delivery therapy might also be useful for pharmacologically active drugs which have demonstrated relative safety but less than optimal clinical results.

Liposomes are one type of drug delivery system. Liposomes are vesicles which are highly ordered assemblages of lipids which contain one or many layers. These structures are used as drug delivery systems because they are biodegradable, and able to carry a variety of lipid- and water-soluble drugs. In order for liposomes to have commercial utility, it is necessary to synthesize these structures such that the number of layers are predictable and the amount of drug present in the carrier is consistent from batch to batch. Further, high drug entrapment is critical for the commercial use of liposomes as drug delivery systems.

Methods of synthesizing liposomes are known in the art. The conventional method for preparing multilayered vesicles was first described by Bangham et al. in the Journal of Molecular Biology 13, 238 (1965). Conventional multilayered vesicles usually entrap only small quantities of drug. Low drug entrapment is caused by the process used to prepare these liposomes. Conventional multilayered vesicles are prepared by adding a drug containing aqueous solution to a film of lipid dried on the bottom of a flask. The aqueous solution penetrates the film thereby hydrating the phospholipids. Hydrated phospholipids spontaneously aggregate to form vesicles. The low drug entrapment is characteristic of this process because of the small surface area between the lipid film and aqueous solution. The drug dissolved in the aqueous solution is not in intimate contact with lipids when they form the liposome membrane.

Papahadjopoulos et al. in U.S. Patent No. 4,235,871 disclose a method of encapsulating biologically active materials in lipid vesicles. This technique circumvented the problem of low drug entrapment by increasing the surface area between the lipid and aqueous solution during liposome membrane formation. The increase in the surface area is accomplished by forming liposomes from water droplets emulsified in an organic phase. Phospholipids in the water and organic solvent emulsion surround the water droplets containing the drug. Subsequent removal of the organic solvent forces the water droplets to coalesce into liposomes. The increase in surface area between the lipid and aqueous phases created by this process insures high drug entrapment within the liposome. This procedure is used to prepare only single layered or oligolayered vesicles on a small scale without the ability to predict beyond one or two layers or amount of drug encapsulation.

Lenk et al. in European Patent No. 92453 disclose a method of preparing multilayer liposomes defined as stable plurilamellar vesicles. Again, predicting the average bilayer number of the liposome and providing high drug entrapment is difficult with this process.

The present invention provides an improved method of preparing multilayered vesicles. Contrary to the process as described above for preparing multilayered vesicles, the present process prepares multilayered liposomes with predictably and consistently the highest drug entrapment known. Further, the present process allows for the control of an average bilayer number per vesicle in the liposome population. The average number of layers for each liposome can be controlled by varying the ratio of lipid to water during the process, and by controlling the water droplet size.

The present invention also provides a new liposome suspension prepared by the present method comprising lipid vesicles having three or more bilayers and x-ray diffraction characteristics as hereinafter specified.

The present invention provides an improved method of encapsulating biologically active materials in lipid vesicles wherein a homogeneous water-in-oil emulsion of a mixture of a lipid, organic solvent, water and the material to be encapsulated is formed, the organic solvent is removed to provide a lipid mixture which is converted to a suspension of vesicles, the improvement comprising controlling the ratio of the amount of lipid to water in the water-in-oil emulsion as defined in Figures 1 through 5 in order to enable one to predict the amount of biologically active material encapsulation in the lipid vesicle and controlling the water drop diameter and amount of lipid as defined in Figure 6 in order to enable one to predict the average number of lipid bilayers in the lipid vesicle.

The present invention also provides a liposome suspension comprising lipid vesicles derived from a water-in-oil emulsion, the average number of bilayers in the vesicles being three or more, said bilayers being electrically neutral, characterized in that the second order peak width of the x-ray diffraction pattern does not increase by more than about 60% when the temperature of the vesicles is increased from about 4°C to about 40°C when the x-ray diffraction data is obtained by pelleting the vesicles; suspending the vesicles in a suitable quantity of phosphate buffered saline; transferring an aliquot of the vesicle suspension into a 1.5 mm quartz capillary tube; sealing the capillary tube; placing the tube in a Cu Kα radiation, double-mirror focusing camera with an order to order resolution of 1500 angstroms having an Elliot GX20 rotating anode generator with a 0.15 x 2.5 mm focal spot; exposing the tube to an x-ray beam at a power of 35 kV and 28 mA; recording the diffraction pattern on Kodak DEF-5 x-ray film with a sample-to-film distance of 300 mm; digitizing film optical densities on a 25 μm raster with an optronics C-4100 film scanner; calculating the average optical density at each radius by performing a circular integration over 25 μm shells; and obtaining radial intensity distribution of each film from the circularly averaged data by subtracting a smooth background curve fitted through the nodes.

Yet another embodiment of the present invention is a liposome suspension comprising lipid vesicles derived from a water-in-oil emulsion, the average number of bilayers in the vesicles being three or more, said bilayers being electrically charged, characterized in that an x-ray diffraction pattern indicates that the lamella repeat distances are substantially uniform when the x-ray diffraction data is obtained by pelleting the vesicles; suspending the vesicles in a suitable quantity of phosphate buffered saline; transferring an aliquot of the vesicle suspension into a 1.5 mm quartz capillary tube; sealing the capillary tube; placing the tube in a Cu Kα radiation, double-mirror focusing camera with an order to order resolution of 1500 angstroms having an Elliot GX20 rotating anode generator with a 0.15 x 2.5 mm focal spot; exposing the tube to an x-ray beam at a power of 35 kV and 28 mA; recording the diffraction pattern on Kodak DEF-5 x-ray film with a sample-to-film distance of 300 mm; digitizing film optical densities on a 25 μm raster with an optronics C-4100 film scanner;

calculating the average optical density at each radius by performing a circular integration over 25 μm shells; and obtaining radial intensity distribution of each film from the circularly averaged data by subtracting a smooth background curve fitted through the nodes.

To illustrate the importance of the relationship between the amount of lipid employed and the amount of water in the emulsion from which liposomes are prepared, a series of experiments were run in which the amount of lipid was held constant at 40 mg, 100 mg or 200 mg while the water was varied from 100 to 1000 μl. The liposomes were prepared in a round bottom flask and no additoinal water was added. The role of the lipid/water relationship in drug entrapment was demonstrated by using $^{14}C$-sucrose in each experiment and measuring the amount of $^{14}C$-sucrose entrapped within the vesicles. The results of these experiments were used to prepare Figures 1-5.

Figure 1 illustrates the relationship of the amount of emulsion-water employed in the process of the invention to the amount of $^{14}C$-sucrose encapsulated within the multilayered vesicle when employing 40 mg of lipid. The graph was based on one trial for each data point.

Figure 2 illustrates the relationship of the amount of emulsion-water employed in the process of the invention to the amount of $^{14}C$-sucrose encapsulated within the multilayered vesicle when employing 100 mg of lipid. The graph was based on the average of the number of trials represented by the numbers in parenthesis.

Figure 3 illustrates the relationship of the amount of emulsion-water employed in the process of the invention to the amount of $^{14}C$-sucrose encapsulated within the multilayered vesicle when employing 200 mg of lipid. The graph is based on the average of the number of trials represented by the number in parenthesis.

Figure 4 represents an entrapment surface diagram comparing percent $^{14}C$-sucrose entrapment, quantity of lipid and emulsion-water. This figure is based on the data illustrated in Figures 1-3.

Figure 5 represents an entrapment surface diagram comparing percent $^{14}C$-sucrose entrapment, quantity of lipid and emulsion-water in different axes than those in Figure 4. This figure is based on the data illustrated in Figures 1-3.

Figure 6 illustrates the amount of phosphatidylcholine required with various size water drops to form an inverted micelle emulsified in excess diethyl ether when employing 300 μl of water in the emulsion. This figure may be used to predict an average bilayer number for multilayered vesicles.

Figure 7 illustrates the dependence of entrapment of $^{14}C$-sucrose on the container in which the process to prepare the present vesicles is conducted. At the gel stage, various amounts of water were added to the preparation.

Figures 8 and 9 compare the x-ray diffraction patterns of vesicles prepared by the present process (MLV-REV) to stable plurilamellar vesicles (SPLV) containing bilayers which were negatively charged. Figure 8 indicates that integral orders of diffraction were obtained for MLV-REV. Similar experiments with SPLV gave a diffuse diffraction pattern as shown in Figure 9. This indicates that MLV-REV have substantially uniform lamella repeat distances, whereas SPLV have lamella repeat distances which are sufficiently heterogeneous to provide diffuse x-ray patterns.

Figures 10-13 compare x-ray diffraction patterns of SPLV with MLV-REV liposomes prepared from solid lipid mixtures of dipalmitoylphosphatidylcholine (DPPC)/cholesterol (CH) (9/1, mol/mol) having bilayers which were electrically neutral wherein heat was applied either during or after lipid assembly into liposome membranes. The Figures indicate that since only integral orders of diffraction were found for SPLV as compared to the diffuse pattern generated for MLV-REV, cholesterol was homogeneously distributed through the bilayers of SPLV, but not the bilayers of MLV-REV.

Figure 14 provides temperature dependent long spacing signatures for neutral vesicles of SPLV, multilayered vesicles as prepared by the procedure of Bangham et al. (MLV), and MLV-REV in both isotonic buffer and water. As the Figure indicates, MLV-REV have a decrease in long spacing when prepared in water as compared to the other two vesicle types. Accordingly, the present method used to assemble lipids into membranes affects the molecular packing of lipids in the membranes in a manner different from the processes used to prepare known vesicles since the present vesicles have a characteristic long spacing signature for lipids dispersed in water as compared to the other two vesicles.

Figure 15 indicates the stability of the vesicles of the present invention as compared to MLV and SPLV vesicles. The Figure provides data indicating the amount of 6-carboxyflurescein (6-CF) which leaks from the vesicles over time based on two trials.

The present method is carried out by combining a lipid, organic solvent, water and biologically active material to provide a mixture. This mixture is next emulsified to give a homogeneous water-in-oil emulsion. The organic solvent is next removed and the resulting gel mixture is finally converted to a suspension of multilayered vesicles which may be isolated by standard procedures.

Lipids suitable for use in the present method are well known in the art and readily available. Useful lipids for use herein include any lipid or lipid mixture containing single or double chain amphiphiles that fulfill the molecular packing requirements of liposomes. The term "liposome" is defined as any lipid particle that contains an internal aqueous compartment. Liposomes may contain either one layer or two layer membranes. See, e.g. Furhop et al. in Journal of the American Chemical Society 106, 4643 (1984), for a description of vesicle formation with a one layered membrane, and Kunitake et al. in Journal of American Chemical Society 102:2, 549-553 (1980) for a description of bilayer vesicles formed from single chain amphiphiles. Exemplary lipids of this type include phosphatidylcholine (PC), dipalmitoylphosphatidic acid (PA), phosphatidylserine (PS), phosphatidylethanolamine, sphingolipids, phosphatidylglycerol (PG), spingomyelin, cardiolipin, glycolipids, gangliosides, cerebrosides, and the like. Of these, phosphatidylcholine is preferred because it is readily available from a variety of sources at a minimal cost relative to other lipids and because it readily forms liposomes. However, the preferred lipids depend on the particular characteristics desired for the intended use of the vesicle and will vary with the particular circumstances involved.

A combination of one or more of the lipids described above may be used as long as the packing requirements for vesicle formation are met. For example, phosphatidylchloline and phosphatidic acid may be combined to provide a useful lipid mixture for use in the present invention. Further, phosphatidylcholine, dipalmitoylphosphatidylcholine, phosphatidic acid may be combined with cholesterol to provide yet another combination contemplated for use herein.

Lipids that lack the ability to form vesicles due to their inability to meet the molecular packing requirements of membranes may be mixed with lipids that form membranes to afford lipids suitable for use in the present method. Examples of these lipids are the steroids, cholesterol (CH), aliphatic amines such as long chain aliphatic amines and carboxylic acids, long chain sulfates and phosphates, dicetyl phosphate, butylated hydroxytoluene, tocopherol, retinoids and isoprenoid compounds. Further, if the biologically active material employed is lipophilic it may be encapsulated by association with the vesicle membrane. When predicting entrapment and bilayer number with lipophilic drugs one must take into account that such drugs may perturb the membranes during lipid assembly.

Lipids, or combinations of one or more lipids, will be either electrically neutral or electrically charged. Lipids which are either electrically neutral or charged are well known in the art. For example, electrically neutral lipids include phosphatidylcholine. Electrically charged membranes include phosphatidylcholine/phosphatidic acid and phosphatidylcholine/phosphatidylserine.

The method of the present invention may be carried out either under an inert atmosphere or under normal atmospheric conditions. The term "inert atmosphere" represents a nonoxidizing atmosphere such as an atmosphere produced by nitrogen, argon or other like inert gases.

In order to prepare lipid vesicles according to the method of the present invention it is first necessary to add a lipid solution to a suitable reaction vessel. Most of the foregoing lipids are commercially available in solution with an organic solvent such as chloroform. Preferably this solvent is removed by vacuum to provide a lipid film on the bottom of the reaction vessel.

When using the present method on a laboratory scale, the reaction vessel used to contain the ingredients may be a round bottom flask, pear shaped flask, test tube or the like, each typical of those routinely used in organic chemistry laboratories. The reaction vessel size is not critical but it will be readily understood that the vessel should not be so large that the emulsion spreads out too thinly on the flask wall or too small to hold the emulsion volume.

The lipid as described above is solubilized in a suitable organic solvent which provides the continuous phase for the emulsion used to form the vesicles. This solvent may be the same as or different than that which is typically employed to solubilize the commercially available lipid. The organic solvents suitable for use herein are preferably chosen so as to form an emulsion with water. Further, the emulsion can be stabilized by combining organic solvents that have a density approximately equal to the density of water so that phase separation of the emulsion is unfavorable. Suitable organic solvents for use herein include those inert solvents such as ethers, esters, alcohols, ketones, aromatic hydrocarbons and aliphatic hydrocarbons, including fluorocarbons and silicons, in which an aqueous phase does not have an appreciable solubility. The solvents may be used alone or in combination.

The biologically active material to be encapsulated by the vesicles prepared by the present method may next be added to the mixture. This material may be any of a variety of compounds which could be contemplated for use including pharmaceuticals such as antibiotics and peptides, and pesticides as well as deoxyribonucleic acid fragments. The compound to be encapsulated may be added to the mixture either in an aqueous solution of a saline buffer, which is preferred, or in a small amount of organic solvent. When a buffer is employed, a variety of such buffers may be employed such as phosphate saline buffer. If an organic solvent is used, water or buffer must be added to the mixture so as to provide aqueous emulsion droplets. A separate biologically active material may not need to be added to the lipid vesicles since the empty vesicles may be biologically active themselves.

The mixture of ingredients thus combined is next emulsified, typically by sonication. In general, a homogeneous emulsion of the character produced by ultrasonic radiation may also be prepared by any number of mechanical means. Ultrasonic radiation may occur over a wide range of temperatures.

The next step of the present method involves removing the organic solvent from the reaction mixture. By removing the organic solvent the present method forms liposomes from water drops emulsified in the organic solvent. Solvent removal may be carried out by either purging the mixture with an inert gas such as nitrogen or argon, or preferably by evaporation under a reduced pressure. When evaporating low boiling solvents such as diethyl ether under reduced pressure, low vacuum is preferred in order to prevent the solvent from flashing. However, it has been determined that removal of the organic solvent by purging with an inert atmosphere must be conducted only in the absence of any sonication of the reaction mixture. Depending on the lipids utilized to form membranes sonication during solvent removal may result in drug loss by mist, perturbation of the membranes during lipid assemble, disruption of the repeat distances being established by the process, and disruption of the lipid mixture in the organic solvent.

Removal of the solvent without sonication during the present method has minimal consequences. Water loss by azeotrope from the emulsion occurs, but membrane perturbation should not occur since the lipids assemble. The amount of water lost during the process of removing the organic solvent is dependent upon the temperature of the emulsion and organic solvent employed. Tables are published which disclose the amount of water lost by various water/organic solvent azeotropes and are readily available to those of ordinary skill in the art. See, e.g., CRC Handbook of Chemistry and Physics, 59th Edition (1978). Water saturated organic solvents may be employed in the present process to compensate for azeotropic water loss. Alternatively, water loss by azeotrope may be compensated for by adding excess water to the aqueous phase of the water and organic solvent emulsion.

It is necessary to employ an amount of lipid in the present process which is in excess of that necessary to cover the surface of the water droplets in the emulsion in order to prepare multilayered vesicles. Lipid in excess of that required to form inverted micelles forms the initial bilayer around the micelles. An inverted micelle results when one layer of lipids completely cover the emulsified water droplet with the polar region of the lipid arranged so as to form the inner portion of the layer. A lipid bilayer exists when a layer of lipid molecules are arranged around an inverted micelle such that the nonpolar, or tail, regions of the molecules are adjacent to each other. Lipid in excess of one complete bilayer forms additional bilayers around the first lamella. Lipids in the emulsion form inverted micelles with the water droplets containing the drug emulsified in the organic phase. As the organic phase is removed, the inverted micelles coalesce into liposomes. The formation of liposomes is believed to occur in sequential steps as follows. Successive lamella are established as a function of time during removal of the organic solvent in the emulsion. As the solvent is removed, the concentration of lipid increases and the lipids begin to coat the inverted micelles. The coating forms a bilayer structure and single layered liposomes are generated. Removing more solvent forces additional excess lipid to form bilayer structures on top of existing liposomes. Bilayer structures are formed on top of existing liposomes because of the small volume of water and large volume of lipid. The exact amount of lipid used in the process will depend on the desired number of bilayers in the vesicle.

Organic solvent remaining in the gel as successive bilayers are laid down allows solutes and solvents to equilibrate across the bilayers, at least during the formation of the initial layers in the gel. Solute and solvent equilibration across the bilayer may not exist in the outermost lamella of the vesicle. Bubbling may occur during the removal of the organic solvent and is believed to facilitate equilibration across the bilayers.

After removing a substantial portion of the organic solvent, the gel-suspension stage results. The gel-suspension stage refers to the emulsion after most of the organic solvent is removed but before excess buffer is added to resuspend the liposomes. Either a gel, suspension or an undefined water/lipid mixture may exist in the gel-suspension stage. Increasing the vacuum at this point may be carried out but is not necessary since it frequently causes the phase to bubble excessively. The appearance of the gel depends upon lipid composition. When the only lipid employed is phosphatidylcholine, a clear gel is obtained. Adding negatively charged lipids, solid lipids or cholesterol to phosphatidylcholine causes the gel to become cloudy or opaque. When only solid lipids are in the mixture, a gel may not be apparent. Typically, however, for these lipid mixtures after most of the organic solvent is removed a gel-suspension stage exists and appears as wet, white precipitated lipids on the bottom of the container. The organic solvent may also affect gel appearance. For example, when isopropyl ether is used to prepare vesicles of the invention composed of dipalmitoylphosphatidylcholine, the emulsion is always cloudy. If diethyl ether is substituted for isopropyl ether, the emulsion is clear, but removing small amounts of the solvent causes the emulsion to become cloudy. The appearance of the emulsion will thus depend on the solubility of the lipids in the water and organic phases used to prepare the vesicles. The volume of the organic solvent will affect the quantity of lipid employed as well.

Gel inversion occurs when the water and organic solvent emulsion inverts and water becomes the continuous phase. The ratio of lipid to water in the emulsion determines the rate at which gel inversion occurs. If the water content of the emulsion is less than that needed to hydrate the lipids when the gel forms, gel inversion will not occur. It is known that approximately 23 μl of water are required to hydrate 100 mg of phosphatidylcholine in the gel-suspension. See Wilkinson et al. in Biophysical Journal 20, 169 (1977). Further, additional water is necessary to solubilize any salt in the buffer, if present, and any biologically active material in the gel-suspension. If this minimum amount of water is not present, gel inversion does not occur. Under these conditions, if bulk water, which is that water which exceeds the water necessary for hydration of the lipid, salt and biologically active material in the gel-suspension, is not available to establish a continuous aqueous phase, liposomes are formed only after the gel is reconstituted with excess buffer. Experimentation has demonstrated that approximately 60% by weight of gel-water to gel-lipid is necessary for the water and organic solvent emulsion to invert smoothly.

During gel inversion water moves from the core of the incompletely formed liposomes to outside the liposomes. Accompanying this water will be the biologically active material sought to be encapsulated, thereby causing the entrapment to decrease in the final liposome population. By adding water, referred to herein as continuous phase water, to the gel, drug entrapment can be optimized by reducing the need for core water to move.

When multilayered vesicles are prepared by the present method water will exist between the repeating bilayers of the vesicle. Water in the repeating bilayers must come from the emulsion droplet core or continuous phase water. Therefore, the diameter of the water droplet is important in predicting the number of bilayers not only for the reasons set forth in Figure 6 which describes the amount of lipid necessary to completely cover emulsified water, but also from the perspective of having sufficient water in the core to provide water between the repeating bilayers. If more bilayers are generated than there is available water in the core to provide adequate quantities of water between the bilayers, the core will collapse, sheets of lipid will result and any predictions of bilayer number will be inaccurate. However, these lipid sheets may be reconstituted into liposomes by rehydrating with water, typically in the form of a buffer.

The gel-suspension which results following removal of most of the organic solvent is next suspended in a suitable buffer solution. Exemplary buffer solutions include those illustrated above such as phosphate saline solution. Typically, the buffer solution is merely added to the reaction flask and agitated throughout the paste, such as by swirling, vortex mixing, or swishing the buffer up and down in the flask with a pasteur pipette. The incorporated aqueous material may be removed if necessary by appropriate and known techniques such as by repeated centrifugations, column chromatography, ion exchange chromatography, dialysis and the like. Finally, the lipid vesicles and their encapsulated contents can be suspended in any isotonic buffer prior to use or administration.

The method of the present invention has been found to be a predictable and convenient method to form vesicles with either a few bilayers, that is from approximately three to five bilayers, to many bilayers, for example, greater than ten. Multilayered vesicles are formed when the emulsified aqueous phase is minimal in relation to the lipid content. One significant aspect of the present method is that it has been determined that by controlling the amount of lipid, the volume of the water, and the water droplet size in the emulsion, the method is capable of generating a liposome with a predictable average number of bilayers and a predictable amount of encapsulated compound. However, it has been determined that the ability to predict bilayer number is possible only if the initial core of the vesicle is not collapsed during the process.

The biologically active material can be contained in the core or in the repeating lamella of the liposome. It has been determined from x-ray diffraction data and computer simulations that regardless of the bilayer number, water spacings typical of repeating lamella, and lipid bilayer thickness in the vesicle, all lamella in multilayered liposome populations entrap volumes similar to completely swollen planar membranes when the core diameter exceeds 2000 angstroms. This is because at this core diameter membrane curvature becomes unimportant with regard to the ability of the lamella to entrap a volume. For example, completely swollen planar bilayers, which have no core, composed of 100 mg of phosphatidylcholine have been found to embibe about 95 μl of water between the bilayers. Since all multilayered liposome populations are heterogeneous with mean diameters near 0.7 μ (7000 angstroms), the inter- lamella trapped volume can be distinguished from the core volume as described. This method of determining the amount of volume trapped within the core is beleived to readily apply to lipid mixtures other than phosphatidylcholine as well.

The method of assembling lipids into liposomes affects entrapment and particle structure. Maximizing entrapment of the biologically active material during the present process depends on maintaining the integrity of the core throughout the process. If the core collapses during the process, entrapment will decrease and the vesicles will have structures that are determined by the lipid mixture and water content in the gel-suspension. Processes for preparing vesicles with a large internal core are capable of entrapping greater amounts of biologically active materials than are processes which result in vesicles with a small internal core. For example, sonicating the lipid emulsion while concurrently evaporating the organic solvent causes core collapse and a lower percent entrapment of available material. In contrast, the present process, which requires evaporating the organic solvent from the emulsion without sonication maintains the size and integrity of the internal core and hence provides a high percent of entrapment. Conventional multilayered vesicles prepared by the procedure of Bangham et al. may have a large core but entrapment is low because the core is only a small fraction of the initial aqueous phase used to prepare the vesicles. In contrast, the cores within the present vesicles contain a large fraction of the original aqueous phase used to prepare the vesicles.

Entrapment of the biologically active material in the present vesicles utilizing lipids with a phase transition temperature is also sensitive to temperature conditions employed during the process. Lipids with a phase transition temperature should be heated above the temperature of the lipid in the membrane with the highest melting point to anneal the membranes. Annealing the membranes may be done either after the vesicles are formed or during the assembly of the lipid into liposome membranes. Unannealed vesicles may contain "cracks", or fissures, which could allow the material contained therein to leak from the vesicles particularly during the process of vesicle purification. For a discussion of the formation and annealing of structural defects in lipid bilayer vesicles, See Lawaczeck et al. in <u>Biochemica</u> <u>Acta</u>. 443, 313-330 (1976). High entrapment with the present process utilizing solid lipids depends on whether the vesicles were heated before washing the suspension as it has been determined that heating before or after lipids assemble into membranes does not significantly affect entrapment. Therefore, annealing must be conducted before washing the vesicles free of unentrapped material, that is, before the vesicles are separated by centrifugation. In contrast, stable plurilamellar vesicles are known not to require heating with solid lipids.

Yet another advantage of the present method is that it is a safe process to entrap toxic and dangerous compounds in multilayered vesicles prepared from water and organic solvent emulsions. Further, as indicated in the description provided herein, the present method permits the ability of entraping a very high percentage of the drug. In view of the foregoing advantages, the present method is particularly well suited for use in the production of liposome vesicles from a commercial standpoint in a large scale industrial setting due to its convenience and predictability.

Figures 1-5 may be used to predict the amount of drug entrapment in a multilayered vesicle in vesicles prepared in a round bottom flask without the addition of continuous phase water. As is readily apparent from Figures 1-3, the amount of emulsion-water required to get an optimum amount of $^{14}C$-sucrose entrapment varies depending on the amount of lipid employed. The entrapment surface diagrams represented by Figures 4 and 5 are interpolations from data generated employing either 40, 100 or 200 mg of lipid in the process. These Figures illustrate the important relationship between the ratio of lipid to water in the emulsion when predicting drug entrapment.

Figure 6 may be used by one of ordinary skill to predict with reasonable certainty the average number of bilayers for each multilayered vesicle in a population of vesicles, and illustrates the importance of water droplet size in predicting average bilayer number. While data has been collected on a small scale, the ability to predict bilayer number is believed possible for large scale processes as well.

Experimental data has shown that the bilayer number in a multilayered vesicle depends on both the lipid concentration and volume of emulsified water present in the mixture. The figure represents data generated from several experiments following the general procedure of Example 1 using a total of 300 µl of water. While it is not yet possible to accurately create a predetermined water droplet size in the emulsion, experimental data has indicated that under the general conditions herein specified average water droplet sizes are approximately 0.5 microns to 1.0 micron in diameter. This diameter is predicted from liposome size distributions obtained from the process disclosed in U.S. Patent No. 4,235,871 which generates liposomes with single or few layers. For water droplets of about 0.5 microns in diameter and larger the total surface area of water drops emulsified in excess diethyl ether is relatively constant. As such, water drops of this diameter may be covered with an appropriate lipid at an amount which is also relatively constant. The asymptotic curve shows little change in the amount of lipid needed to cover 300 µl of emulsified water droplets having a diameter between 0.5 and 0.9 microns. For example, approximately 6 mg of lipid is needed to produce an inverted micelle when the water droplets have an average diameter of about 0.5 microns as compared to 3.7 mg of lipid which is needed to provide an inverted micelle when the water droplets have an average diameter of about 0.9 microns.

The amount of emulsion-lipid and emulsion-water are important to the type of liposome formed. A heterogeneous population of emulsion water drops between 0.5 and 1.0 microns in diameter can generate a heterogeneous distribution of liposomes. The liposomes would be predominantly single or multilayered with an average number of bilayers depending on the ratio of emulsion-lipid to emulsion water.

The average number of bilayers for each vesicle may be determined by the following formula:

$$\text{Amount of Lipid in the Emulsion} \div \left[\frac{\mu l \text{ of total water} - \mu l \text{ of water lost by azeotrope}}{\mu l \text{ of total water}}\right.$$

X twice the amount of lipid required to completely cover a water droplet of a specified diameter] = average number of bilayers for each vesicle

For example, when employing a water droplet size which is 0.56 microns in diameter, 100 mg of lipid, 300 μl of total water and 10 ml of diethyl ether, the following calculation may be made:

$$100 \text{ mg} \div \left[\frac{300\ \mu l - 100\ \mu l}{300\ \mu l} \text{ X } 12 \text{ mg/bilayer}\right]$$

= approximately 12 bilayers for each vesicle. The amount of water lost by azeotrope for diethyl ether is a known constant. Better estimates of bilayer number in the final liposome population will be obtained when cores can be controlled and when one takes into consideration the exact volume of lipid in each repeating lamella as the particle changes size. Further, when using lipids other than phosphatidylcholine, the area per molecule of the lipid must be taken into account to calculate the amount of lipid needed to cover the emulsion droplet. In view of the foregoing, the amount of water in the emulsion, the amount of water in the gel-suspension, sonication time and sonication power all influence the type of liposome which will be formed. For example, increasing the amount of sonication to produce small emulsified water drops causes a smaller liposome diameter, a smaller number of bilayers, a smaller inside core diameter and a lower drug entrapment.

The highest percentage of entrapment of the biologically active material in the present process is dependent upon the volume of emulsion water, the type and amount of lipid, and the container used to prepare the vesicles. It has been found that without adding continuous phase, or bulk, water to the mixture, the present process entraps a higher percentage of biologically active material when a test tube is used than when a round bottom or pear shaped flask is used. Further, it has been found the use of 100 mg of phosphatidylchloine entraps more efficiently than 150 mg of the same lipid. While adding small volumes of continuous phase water to vesicles prepared in a test tube has little effect on entrapment, the addition of 300 µl of such water decrease entrapment about 10% when using 100 mg of phosphatidylcholine. Increasing the drug concentration in the emulsion water effects the amount of drug entrapped. Thus, optimum entrapment in the present process depends on optimizing experimental conditions.

It has also been determined that the percent of the biologically active substance entrapped by the present process is dependent upon the type of container in which the process is conducted. In Figure 7, data is presented on percent entrapment of $^{14}$C-sucrose in both a 25 mm x 175 mm test tube and a 100 ml round bottom flask. The emulsion was made with 10 ml of ether, 0.3 ml of PBS containing 1 mg/ml sucrose and either 100 mg or 150 mg of phosphatidylcholine. At the gel stage, various amounts of continuous phase water were added. Entrapment values were corrected for lipid recovery and reflect the mean ± s.d. for n>3. As the Figure indicates, higher entrapment occurred when the vesicles were prepared in a test tube. Higher entrapment may be the result of a larger average core in the liposomes contained in the final population. It may also be do to the decreased ability of the drug to escape from the liposomes during gel inversion when continuous phase water is added to prevent core water from becoming extra-liposomal water.

The present invention also provides new liposome vesicles prepared by the process of the present invention.

As noted above, and as demonstrated by the data provided hereinafter, the method of preparing liposomes affects particle structure. For example, sonicating the emulsion during lipid assembly while concurrently evaporating the organic solvent facilitates lipid equilibration throughout the lamella, but disturbs the incompletely formed bilayers in the particle. This sonication causes negatively charged vesicles to elicit hetereogeneous repeat distances in the final liposome population. However, evaporating the solvent from the emulsion in the absence of sonication, as is required by the present process, generates homogeneous lamella in the final liposome population of negatively charged vesicles. Homogeneous repeat distances of electrically charged vesicles are elicited by the present invention because intralamellar water spacings are not disrupted during liposome formation. For some lipid mixtures, the absence of sonication during lipid assembly may form liposomes with an inhomogeneous distribution of lipid molecules throughout the liposome. Thus, an assymetric distribution of lipid between individual lamella in the liposome may exist in vesicles prepared by the present invention. An assymetric lipid distribution in the liposome may affect long spacing and permeability rates.

X-Ray diffraction data discussed hereinafter was obtained as follows. The vesicles were pelleted by microfuge, supernatants discarded and 25 to 50 microliters of PBS added to the pellet. With a syringe, an aliquot of the concentrated liposome pellet was pipetted into the center of a 1.5 mm quartz capillary tube and sealed. The vesicles tested were about 125 mg/ml. Diffraction data were collected using Cu K$\alpha$ radiation, a double-mirror focusing camera with an order-to-order resolution of 1500 angstroms, and an Elliot GX20 rotating anode generator with a 0.15 x 2.5 mm focal spot. Exposure times varied depending on the sample and were conducted at an x-ray power of 35 kV and 28mA. Diffraction patterns were recorded on Kodak DEF-5 x-ray film with a sample-to-film distance of 300 mm. An optronics C-4100 film scanner was used to digitize film optical densities on a 25 μm raster. The center of each diffraction pattern was determined using the symmetry of the pattern. The average optical density at each radius was calculated by performing a circular integration over 25 μm shells. The radial intensity distribution of each film was obtained from these circularly averaged data by subtracting a smooth background curve fitted through the nodes.

The process used to prepare liposomes is thus important in determining the structures in the final liposome population. In this regard, processes used to prepare liposomes can create lamella with irregular repeating lamella. Lipid mixtures that allow long spacing to vary substantially are most susceptible to irregular distances in the repeating lamella of the final liposome population. Two lipid mixtures that allow repeat distances to vary several angstroms are DPPC/CH (9/1, mol/mol) and PC/PA (9/1, mol/mol) or PC/PS (9/1, mol/mol). Negative charges imparted on the membrane by PA and PS allow the membranes to separate well beyond the approximate 30 angstrom separation expected from neutral PC bilayers. Long spacing increases 15 angstroms between DPPC and DPPC/CH (9/1, mol/mol) liposomes. Ladbrooke et al. Biochin. Biophys. Acta 333-340 (1968). Long spacing of a DPPC/CH mixture is thus very sensitive to the membrane concentration of CH, particularly near 10 mole percent cholesterol. If processes used to make multilayered liposomes from the mixtures above create inhomogeneous lipid distributions in the final vesicle population, one can expect inhomogeneous long spacings. If all of the repeating distances are irregular then one will not obtain a diffraction pattern from the vesicles.

X-ray diffraction studies of vesicles containing bilayers which are electrically charged demonstrate that hetereogeneous repeat distances of bilayers can occur depending on the method used to assemble the lipids. Figures 8 and 9 compare the x-ray diffraction patterns of vesicles prepared by the present process with SPLV containing bilayers which are negatively charged. Conventional MLV gave the same pattern as SPLV. Figure 8 indicates that integral orders of diffraction were obtained with vesicles of the present invention prepared from phosphatidylcholine/phosphatidic acid (9/1, mol/mol) and phosphatidylcholine/phosphatidylserine (9/1, mol/mol) following a two hour exposure at about 26°C. In contrast, Figure 9 indicates that SPLV prepared with phosphatidylcholine/phosphatidylserine (9/1, mol/mol) gave a diffuse diffraction pattern which was obtained after fifteen hours exposure time at about 26°C. Additional determinations were made wherein the vesicles eliceted no diffraction pattern. Accordingly, SPLV have lamella repeat distances which are heterogeneous, as evidenced in that diffraction patterns are absent, or long x-ray diffraction exposure times are needed to obtain diffuse patterns. The Figures therefore indicate that the arrangement of bilayers in the vesicles of the present invention is substantially uniform when the bilayers are electrically charged. These substantially uniform lamella repeat distances occur only for the present vesicles and not for conventional MLV vesicles or stable plurilamellar vesicles.

Figures 10-13 provide x-ray diffraction patterns of vesicles prepared from solid lipid mixtures wherein the vesicles were either prepared at about 26°C and the gel-suspension was heated at 50°C for one hour, or the lipids were assembled at 50°C and the gel suspension was maintained at about 26°C for about five minutes followed by vesicle purification. As can be seen from the Figures, integral orders of diffraction were found for SPLV, whereas assymetric diffraction patterns were obtained for the vesicles of the present invention. This indicates that cholesterol is homogeneously distributed throughout the lamella of SPLV but not the vesicles of the present invention.

Assymetric x-ray diffraction patterns of vesicles of the present invention composed of dipalmitoylphosphatidylcholine/cholesterol (9/1, mol/mol) are dependent upon the history of the sample. Occasionally symmetric peaks and integral orders of diffractions were obtained with the present vesicles having the aforementioned lipid compositions. Accordingly, sufficient mixing of the present process may not occur depending on the experiment conditions.

The manner in which lipids are assembled affects the molecular packing in liposome membranes as well. In comparing conventional MLV vesicles to SPLV vesicles a characteristic temperature dependent long spacing for each of these vesicles has been reported. This feature of the vesicles was described as a long spacing signature (LSS) capable of distinguishing SPLV from conventional MLV vesicles. Gruner et al. Biochemistry 24, 2833-2842 (1985). Figure 14 shows the LSS for conventional MLV, MLV-REV and SPLV vesicles prepared in isotonic buffer and water. The Figure confirms that SPLV can be distinguished from MLV because SPLV have a lamella repeat distance that remains relatively constant over the temperature range of about 4° to about 40°C whereas conventional MLV elicit a substantial decrease in long spacing. MLV-REV vesicles prepared in buffer are similar to SPLV as expected because both MLV-REV and SPLV are prepared from water-in-oil emulsions.

The difference in long spacing signatures of SPLV and MLV vesicles prepared in buffer has been attributed to a homogeneous solute distribution for SPLV compared to a heterogeneous solute distribution for MLV. This hypothesis was given because when SPLV and MLV vesicles are prepared in the absence of solutes the long spacing signatures are identical. It has been determined that MLV and SPLV vesicles have similar long spacing signatures when the vesicles are prepared in water. However, the vesicles of the present invention have a characteristic decrease in long spacing. The long spacing in the present vesicles prepared from phosphatidylcholine decrease from 59.5 angstroms at 3°C to 56.6 angstroms at 40°C. Since the long spacing signatures of the present vesicles obtained when the lipids were dispersed in water do not coincide with conventional MLV and SPLV liposomes, the hypothesis that SPLVs are osmotically balanced and MLV contain a significant internal solute gradient is questionable. Although a salt gradient may exist, it may not cause the temperature dependent long spacing changes observed for conventional MLV when the vesicles are prepared in buffer. In addition to osmotic factors, the long spacing signature is sensitive to the method used to assemble the lipids into liposome membranes.

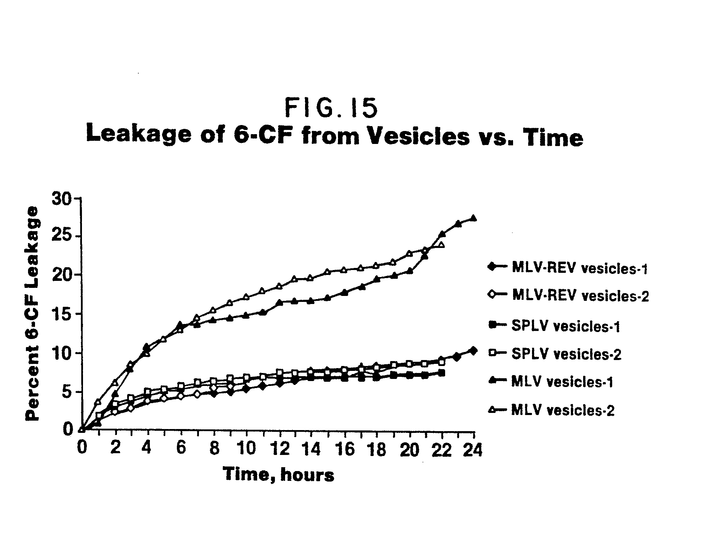

Figure 15 indicates that the vesicles of the present invention release much less 6-CF than conventional vesicles and are hence much more stable than those vesicles. The procedure employed to determine 6-CF leakage is described by Pidgeon et al., in Photochemistry and Photobiology Vol. 37, No. 5, 491-494 (1983).

The vesicles of the present invention, when containing bilayers which are electrically neutral, possess a second order peak width of an x-ray diffraction pattern obtained by the method hereinbefore described which does not increase by more than about 60% when the temperature of the vesicles is increased from about 4°C to about 40°C. This data is presented below in Table I, and is compared with SPLV prepared with the same lipid, for vesicles which are either 1 day old or 14 days old.

Table 1

Second Order Peak Width of
MLV-REV and SPLV with Neutral Bilayers (centimeters)

| Age of Vesicles (Days) | Vesicle | Temperature 4°C | 26°C | 40°C | Increase in Peak width from 4°C to 40°C (Percent) |
|---|---|---|---|---|---|
| 1 | MLV-REV | 1.0 | 1.25 | 1.4 | 40.0 |
| | SPLV | 0.65 | 0.80 | 1.25 | 92.3 |
| 14 | MLV-REV | 0.90 | 1.10 | 1.25 | 38.9 |
| | SPLV | 0.65 | 1.20 | 1.75 | 169.2 |

The following examples illustrate the vesicles of the present invention, and the present method of preparing the vesicles. The examples are not intended to limit the scope of the present invention in any respect and should not be so construed.

Examples 1 and 2 below illustrate encapsulation studies of neutral liposomes composed of egg phosphatidylcholine. Phospholipid purity was periodically evaluated by thin layer chromatography whereby about 2 μmol of lipid was chromatographed on silica gel plates eluting with chloroform:methanol:water (65:25:4, v:v:v). Phospray from Supelco, Bellefonte, Pennsylvania was used to detect the presence of phospholipid phosphate.

## Example 1

A 100 ml round bottom flask was charged with 100 mg of egg phosphatidylcholine (Avanti Polar Lipids, Inc., Birmingham, Alabama) dissolved in chloroform. The solvent was evaporated under reduced pressure at about 40°C for 5 minutes and 10 ml of diethyl ether was added thereto. In a separate container radiolabeled $^{14}C$-inulin (2.4 mci/g from New England Nuclear, Boston, Massachusetts) was combined with 0.3 ml of phosphate buffered saline. The compound containing solution was added to the flask which was agitated in a water bath sonicator for 2 minutes under an argon atmosphere at about 25°C. The diethyl ether was evaporated *in vacuo* to provide the gel-suspension stage. Ten milliliters of phosphate buffered saline were added to the round bottom flask in order to suspend the gel-suspension in the buffer solution. Suspension was carried out by either swirling or vortex mixing the buffer up and down in the flask with a pasteur pipette. The liposomes thus prepared were isolated by transferring the mixture to a test tube which was subsequently centrifuged at about 10,000 rpm for about 10 minutes. The pellet thus prepared was resuspended in 10 ml of phosphate buffered saline in order to wash the liposomes. The liposomes were again collected by centrifugation. The resultant liposomes were analyzed to indicate the presence of 65 ± 15% of liposome associated inulin. The lipid recovery was 97% ± 3.5%. Encapsulation efficiency was 1.59 ± 0.33 µl trapped volume as water per µmol of lipid present.

## Example 2

Following the general procedure outlined in Example 1 but replacing the radiolabeled $^{14}C$-inulin with radiolabeled $^{14}C$-sucrose (360 mci/mmol from ICN, Irvington, California) the following results were obtained. The percent of liposome associated with sucrose was 52.2 ± 14%. Lipid recovery was 92.4%. The encapsulation efficiency was 1.14 ± .14 µl trapped volume as water per µmol lipid. The values for sucrose encapsulation are the mean ± sd for 5 determinations.

Examples 3, 4 and 5 set forth below illustrate encapsulation studies using negatively charged liposomes.

Example 3

To a 100 ml round bottom flask was added 100 mg of a 9:1 (mol:mol) mixture of a combination of phosphatidylcholine:dipalmitoylphosphatidic acid (Sigma Chemical Company, St. Louis, Missouri) dissolved in chloroform. The solvent was evaporated under reduced pressure at 40°C for approximately 5 minutes and 10 ml of diethyl ether was added thereto. Radiolabeled $^{14}C$-inulin as an aqueous space marker was combined in a small tube with 0.3 ml of phosphate buffered saline. The resulting solution was added to the round bottom flask and the flask was sonicated for 2 minutes under argon at 25°C in a water bath sonicator. The diethyl ether was evaporated under reduced pressure to provide a gel-suspension. Ten milliliters of phosphate buffered saline were added to the suspension in the flask and the gel-suspension was suspended in the buffer solution by swirling the buffer within the flask with a pasteur pipette. The mixture was transferred to a centrifugation tube for centrifugation at 10,000 rpm for 10 minutes. The resulting pellet was suspended in 10 ml of phosphate buffered saline and again collected by centrifugation. The resulting liposomes were analyzed to indicate the presence of 26% liposome associated inulin with a lipid recovery of 82%. Encapsulation efficiency was 0.748 µl trapped volume as water per µmol of lipid.

Example 4

A 100 ml round bottom flask was charged with a solution of 100 mg of lipid consisting of phosphatidylchloline:dipalmitoylphosphatidic acid:cholesterol (Sigma Chemical Company, St. Louis, Missouri) in a molar ratio of 5:1:4 dissolved in chloroform. The solvent was evaporated under reduced pressure at 40°C for approximately 5 minutes and 10 ml of diethyl ether was added thereto. In a separate container radiolabeled $^{14}C$-sucrose was dissovled in 0.3 ml of phosphate buffered saline. The resulting solution was added to the flask, and the flask was sonicated for 2 minutes under an argon atmosphere at 25°C in a water bath sonicator. The diethyl ether was removed in vacuo to provide a gel-suspension. Ten milliliters of phosphate buffered saline were added to the flask and, by swirling the buffer around in the flask, the gel-suspension was suspended. The mixture was transferred to a test tube whereby the liposomes were collected by centrifugation at 10,000 rpm for 10 minutes. The resulting pellet was isolated and resuspended in 10 ml of physiologic saline in order to wash the liposomes. The pellet was again generated by centrifugation. The liposomes were analyzed to indicate the presence of 25 ± 2% liposome associated sucrose resulting in a lipid recovery of 87 ± 5.3%. The encapsulation efficiency was 0.522 ± 0.036 μl trapped volume as water per μmol of lipid. The value reported herein were determined by taking the mean ± sd for 3 determinations.

Example 5

Following the general procedure outlined in Example 4 but replacing the 100 mg quantity of lipid with 20 mg of the same lipid the following results were obtained. The liposome associated sucrose was 12 ± 5.6% adding a lipid recovery of 91 ± 3%. The encapsulation efficiency was 1.22 ± 0.6 μl trapped volume as water per μmol of lipid. Reported values were obtained by taking the mean ± sd for 3 determinations.

The following Examples indicate the amount of $^{14}C$-sucrose entrapped with a variety of lipid mixtures both with and without the addition of continuous phase water. This data is presented in Table II below.

Table II

Entrapment of $^{14}$C-Sucrose

| Lipid Mixture | mg/mg | Mole % PC | % Entrapped: Continuous Phase Water 0.0 µl | % Entrapped: Continuous Phase Water 100 µl |
|---|---|---|---|---|
| PC | 100 | 100 | 90.5 ± 4.2 | 90.9 ± 5.3 |
| PC/PS | 100/8.3 | 93 | 83.9 | 86.7 |
| PC/PA | 100/4 | 95 | 84.2 ± 5.0 | 90.9 ± 2.2 |
| PC/PG | 100/10 | | 60.8 ± 6.0 | 90.0 ± 1.7 |
| PC/CH | 100/10 | 83 | 57.5 ± 3.7 | 91.1 ± 2.2 |
| PC/CH | 94/15 | 75 | 87.4 ± 2.0 | 83.8 ± 6.6 |
| PC/CH | 80/20 | 66 | 70.0 ± 17.5 | 64.8 ± 10.9 |

The following examples were conducted employing different materials and phosphatidylcholine as the lipid while carrying out the process in a test tube. These results are set forth in Table III below.

Table III

Entrapment of Various Materials With Test Tube

| | % Entrapped | |
|---|---|---|
| | Continuous Phase Water (μl) | |
| | 0 | 100 |
| α-aminobutyric acid | 52.3 | 61.7 |
| tobramycin | 74.2 | 83.7 |
| poly(I)·poly(C) | 83.5 | 65.8 |
| tyrosine | 65.2 | 70.3 |

Vesicles of the invention were also prepared with solid lipids. Dipalmitoylphosphatidylcholine was used as a model compound to study drug entrapment in the present vesicles prepared from lipids with a phase transition temperature. The phase transition temperature of dipalmitoylphosphatidylcholine is 41.5°C, and diethyl ether has a boiling point of 34.6°C at 760 mm. Since liposomes must be heated above the phase transition temperature of the highest melting lipid in the liposome membrane the present vesicles composed of dipalmitoylphosphatidylcholine or dipalmitoylphosphatidylcholine/cholesterol were prepared by two methods. The first method involved heating the liposomes after emulsion ether was removed, that is, after the lipids assembled into membranes, whereas the second method involved heating the liposomes during lipid assembly into membranes. The first method is identical to the procedure used for fluid liposomes described above except the gel-suspension was heated to 50°C for one hour. For the second method diisopropyl ether, having a boiling point of 68°-69°C at 760 mm, was substituted for diethyl ether, and the present process was performed at 50°C instead of 30°C. For the second method diisopropyl ether was evaporated in two stages and, as with the first method, when most of the organic solvent was removed, a gel-suspension formed. Dipalmitoylphosphatidylcholine/cholesterol mixtures were reconstituted with either 1 ml or 10 ml of PBS at about 25°C. Vesicle purification and drug entrapment were performed by the general method described above. Since about 90 µl of water is lost from a diethyl ether/water azeotrope, diisopropyl ether was saturated with water by equilibrating overnight at 25°C with 1/1 (v/v) mixtures of diisopropyl ether/water. The entrapment data is set forth below in Table IV.

Table IV

Entrapment With Solid Lipids (Percent)

| DPPC/CH mg/mg | Mole % CH | Diethyl Ether/PBS 10 ml/0.3 ml | Saturated Diisopropyl Ether/PBS 10 ml/0.5 ml |
|---|---|---|---|
| 100/0 | 0 | 31.95 ± 4.8 | 61.9 ± 2.95 |
| 94/6 | 10 | 49.7 ± 17.3 | 49.0 ± 0.5 |
| 88/12 | 17 | 29.1 ± 7.1 | 44.3 ± 5.9 |
| 81/19 | 33 | 31.7 ± 7.6 | 55.5 ± 2.8 |
| 74/26 | 40 | 42.8 ± 9.1 | 59.4 ± 1.7 |
| 65/35 | 52 | 31.7 ± 14.4 | 57.3 ± 4.3 |

Claims

1. A method of encapsulating biologically active materials in lipid vesicles wherein a homogeneous water-in-oil emulsion of a mixture of a lipid, organic solvent, water and the material to be encapsulated is formed, the organic solvent is removed to provide a lipid mixture which is converted to a suspension of vesicles, characterized in that:

the ratio of the amount of lipid to water in the water-in-oil emulsion is controlled as defined in Figures 1 through 5 in order to enable one to predict the amount of biologically active material encapsulation in the lipid vesicle and controlling the water drop diameter and amount of lipid as defined in Figure 6 in order to enable one to predict the average number of lipid bilayers in the lipid vesicle.

2. A method of encapsulating biologically active materials in lipid vesicles wherein a homogeneous water-in-oil emulsion of a mixture of a lipid, organic solvent, water and the material to be encapsulated is formed, the organic solvent is removed to provide a lipid mixture which is converted to a suspension of vesicles, characterized in that:

the ratio of the amount of lipid to water in the water-in-oil emulsion is controlled as defined in Figures 1 through 5 in order to enable one to predict the amount of biologically active material encapsulation in the lipid vesicle.

3. A method of encapsulating biologically active materials in lipid vesicles wherein a homogeneous water-in-oil emulsion of a mixture of a lipid, organic solvent, water and the material to be encapsulated is formed, the organic solvent is removed to provide a lipid mixture which is converted to a suspension of vesicles, characterized in that:

the water drop diameter and amount of lipid in the water-in-oil emulsion is controlled as defined in Figure 6 in order to enable one to predict the average number of lipid bilayers in the lipid vesicle.

4. A liposome suspension comprising lipid vesicles derived from a water-in-oil emulsion, the average number of bilayers in the vesicles being three or more, said bilayers being electrically neutral, characterized in that the second order peak width of the x-ray diffraction pattern does not increase by more than about 60% when the temperature of the vesicles is increased from about 4°C to about 40°C when the x-ray diffraction data is obtained by pelleting the vesicles; suspending the vesicles in a suitable quantity of phosphate buffered saline; transferring an aliquot of the vesicle suspension into a 1.5 mm quartz capillary tube; sealing the capillary tube; placing the tube in a Cu Kα radiation, double-mirror focusing camera with an order to order resolution of 1500 angstroms having an Elliot GX20 rotating anode generator with a 0.15 x 2.5 mm focal spot; exposing the tube to an x-ray beam at a power of 35 kV and 28 mA; recording the diffraction pattern on Kodak DEF-5 x-ray film with a sample-to-film distance of 300 mm; digitizing film optical densities on a 25 μm raster with an optronics C-4100 film scanner; calculating the average optical density at each radius by performing a circular integration over 25 μm shells; and obtaining radial intensity distribution of each film from the circularly averaged data by subtracting a smooth background curve fitted through the nodes.

5. A liposome suspension comprising lipid vesicles derived from a water-in-oil emulsion, the average number of bilayers in the vesicles being three or more, said bilayers being electrically charged, characterized in that an x-ray diffraction pattern indicates that the lamella repeat distances are substantially uniform when the x-ray diffraction data is obtained by pelleting the vesicles; suspending the vesicles in a suitable quantity of phosphate buffered saline; transferring an aliquot of the vesicle suspension into a 1.5 mm quartz capillary tube; sealing the capillary tube; placing the tube in a Cu $K\alpha$ radiation, double-mirror focusing camera with an order to order resolution of 1500 angstroms having an Elliot GX20 rotating anode generator with a 0.15 x 2.5 mm focal spot; exposing the tube to an x-ray beam at a power of 35 kV and 28 mA; recording the diffraction pattern on Kodak DEF-5 x-ray film with a sample-to-film distance of 300 mm; digitizing film optical densities on a 25 μm raster with an optronics C-4100 film scanner; calculating the average optical density at each radius by performing a circular integration over 25 μm shells; and obtaining radial intensity distribution of each film from the circularly averaged data by subtracting a smooth background curve fitted through the nodes.

FIG.1

**Multilayered Vesicles Prepared From 40 mg of Lipid**

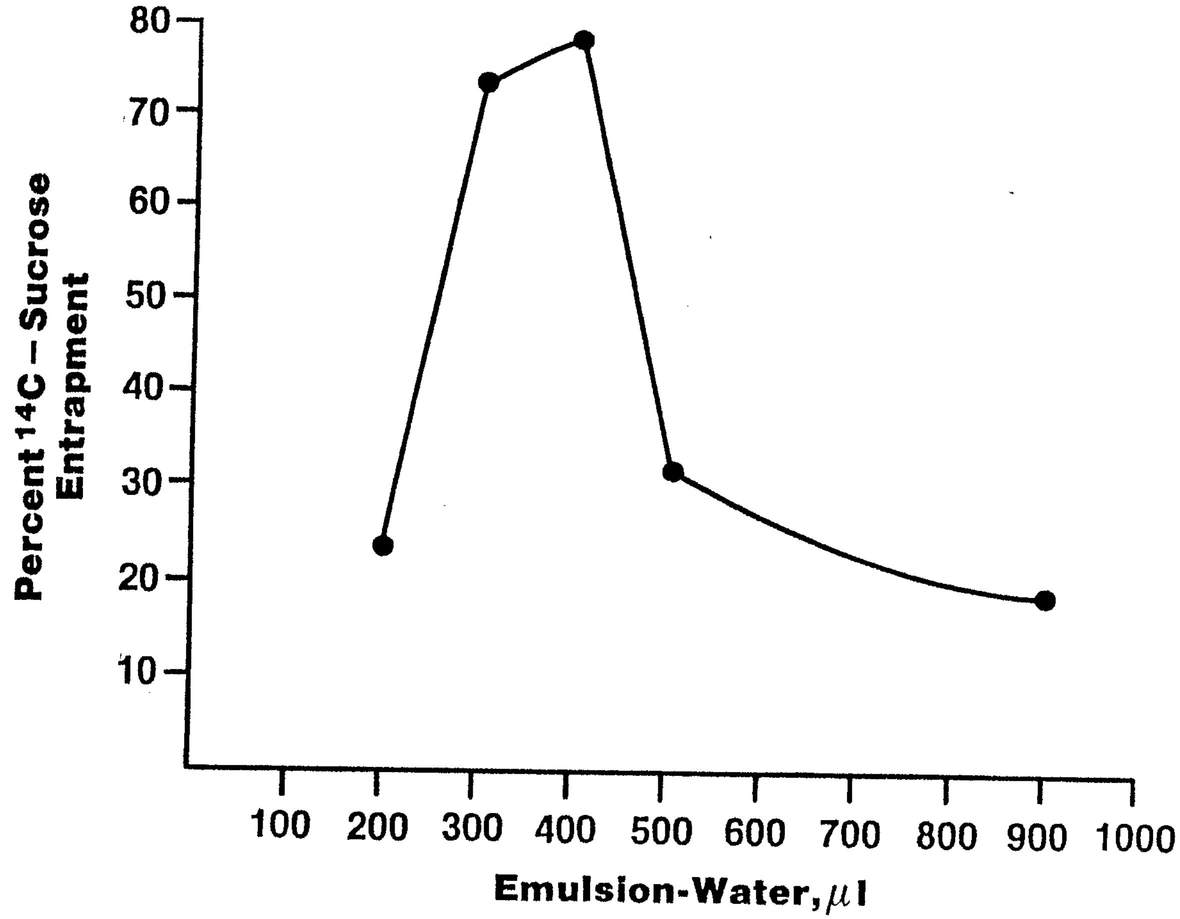

FIG.2

**Multilayered Vesicles Prepared From 100 mg of Lipid**

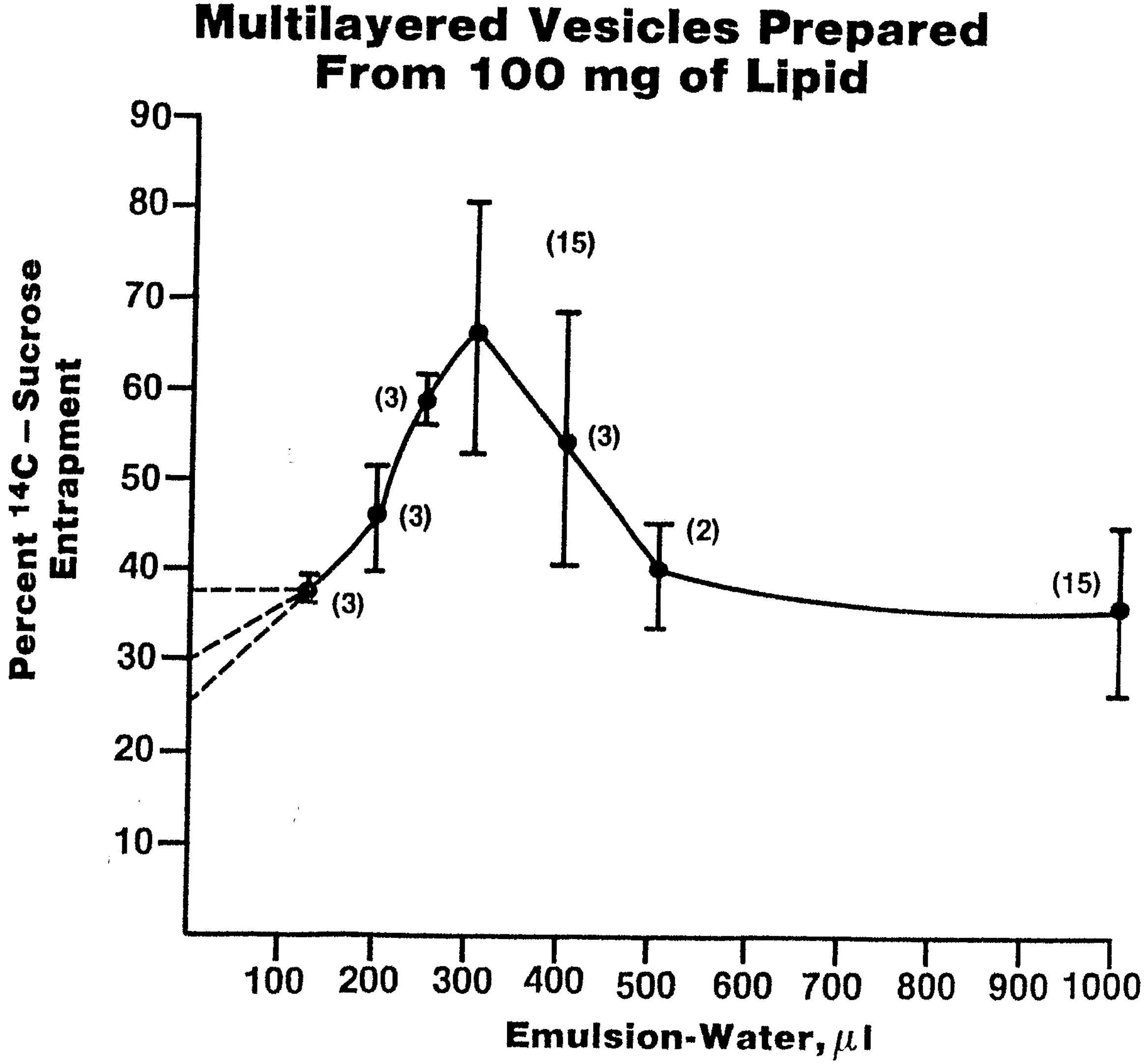

FIG.3

**Multilayered Vesicles Prepared From 200 mg of Lipid**

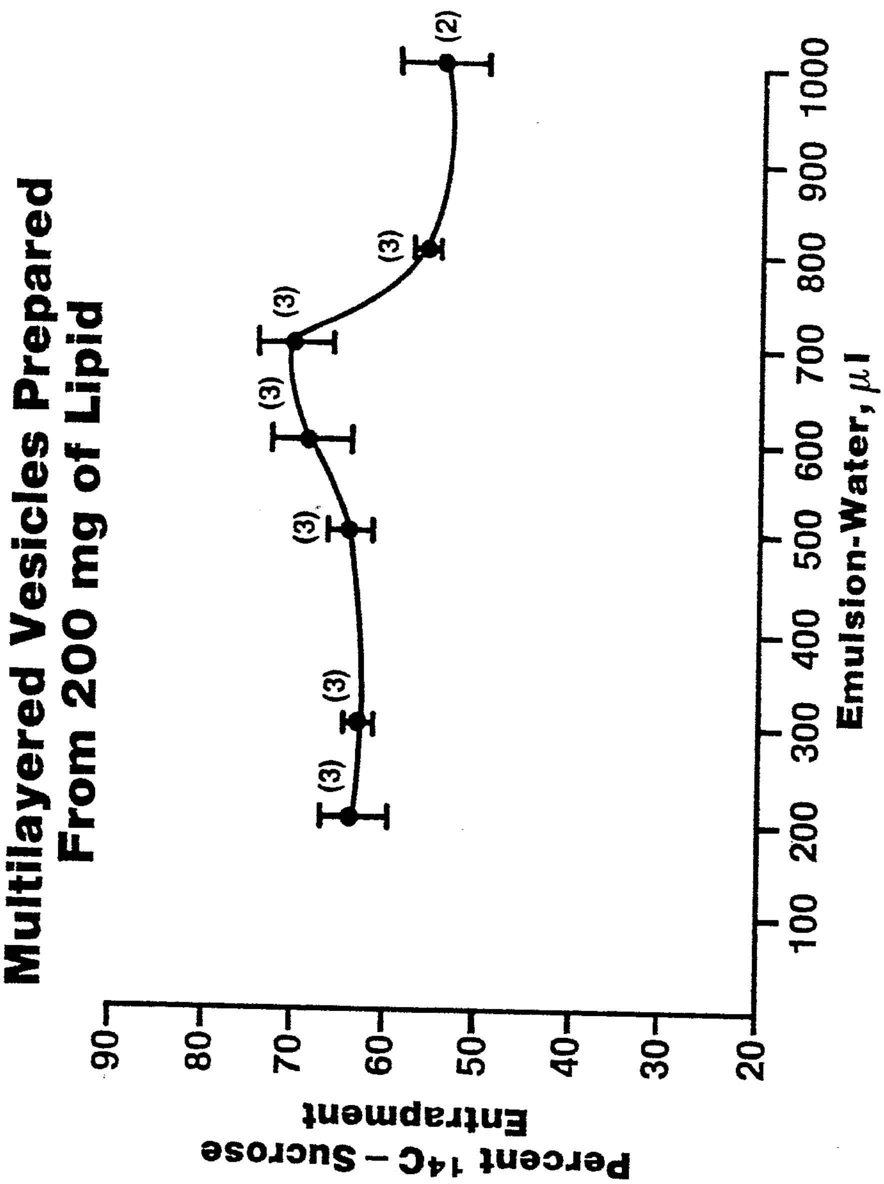

FIG. 4

**Entrapment Surface Diagram**

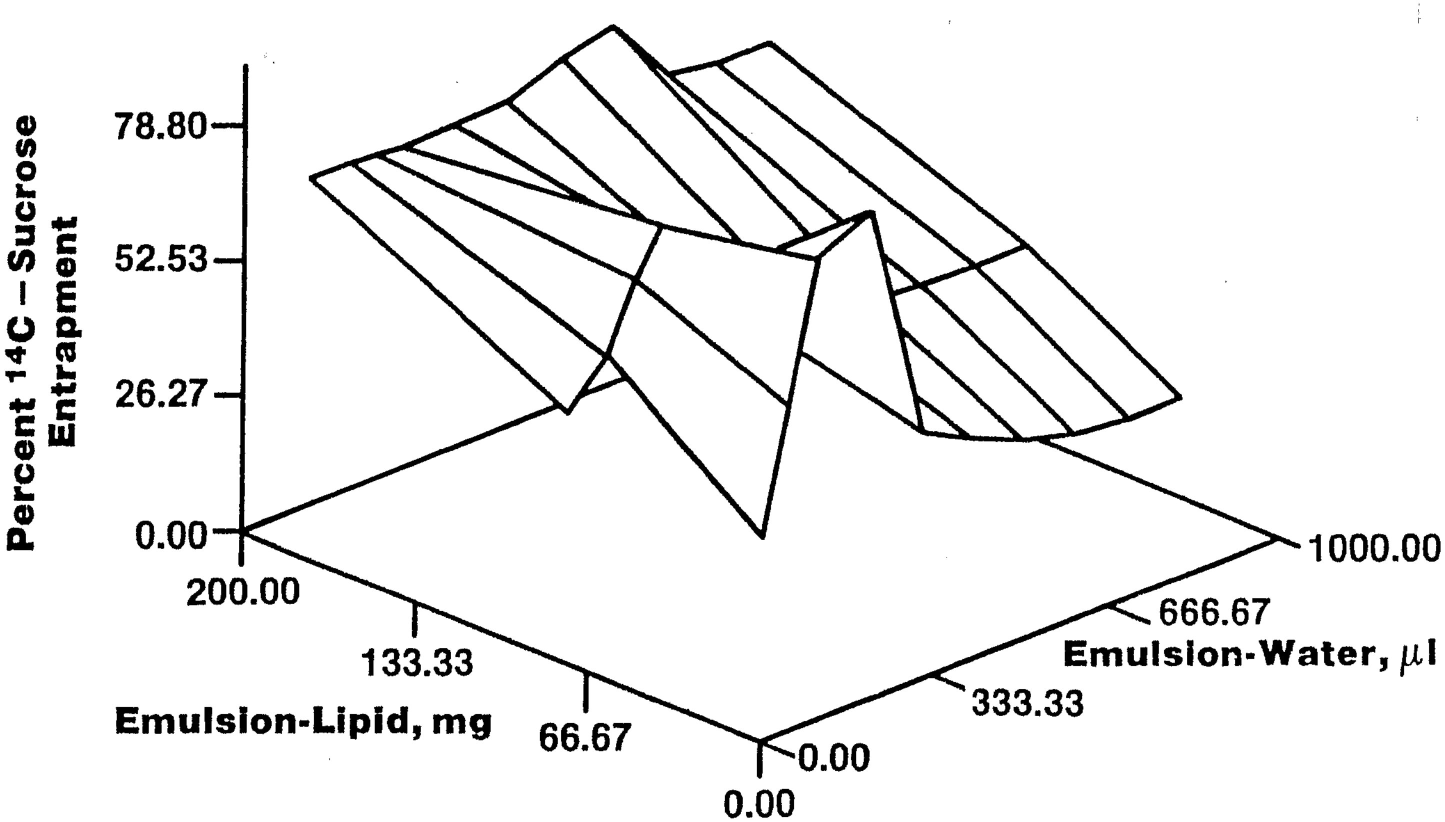

FIG. 5

**Entrapment Surface Diagram**

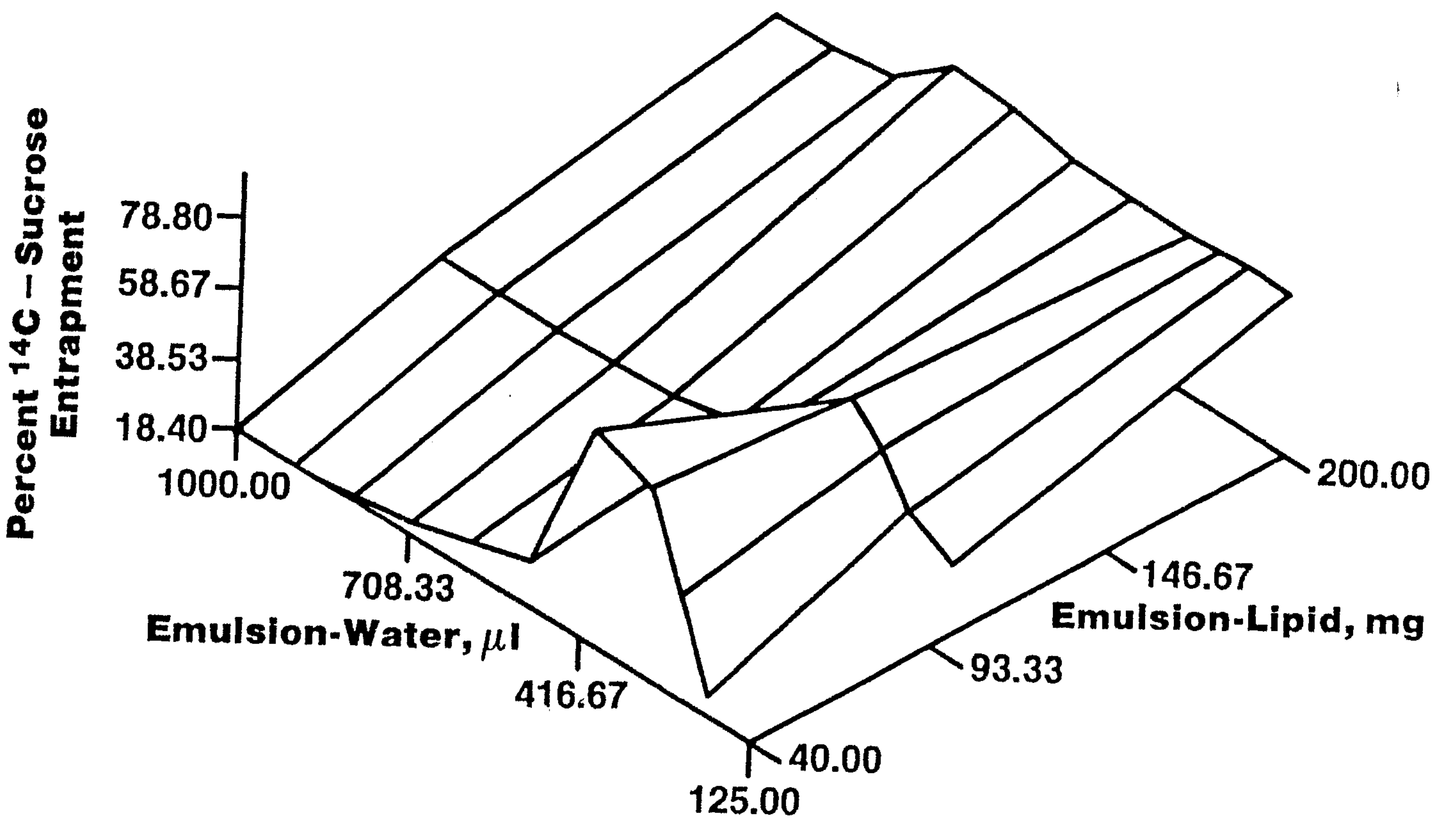

FIG. 6

**Phosphatidylcholine Required to Completely Cover Emulsified Water**

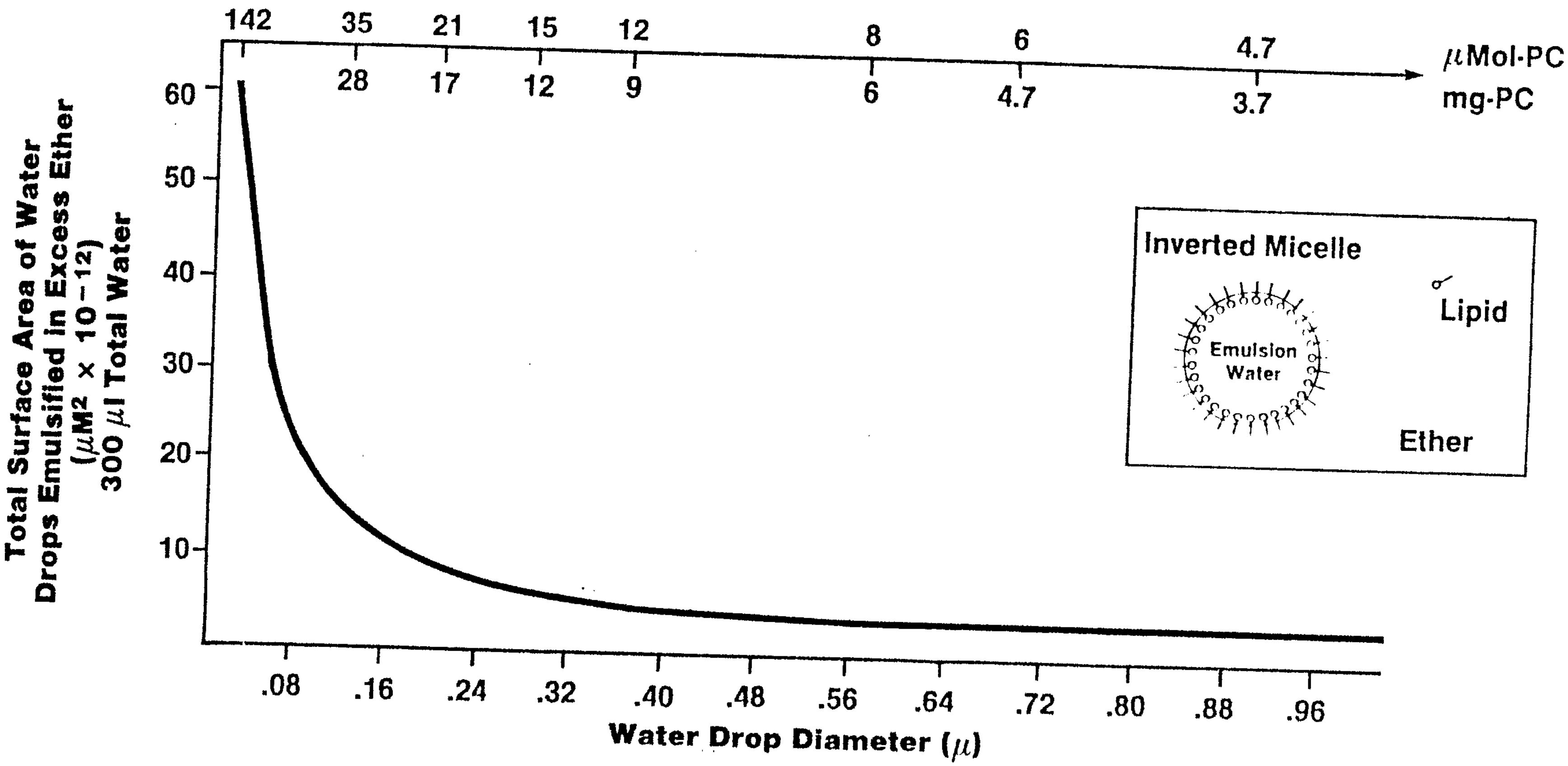

FIG.7

**Dependence of Entrapment on Container**

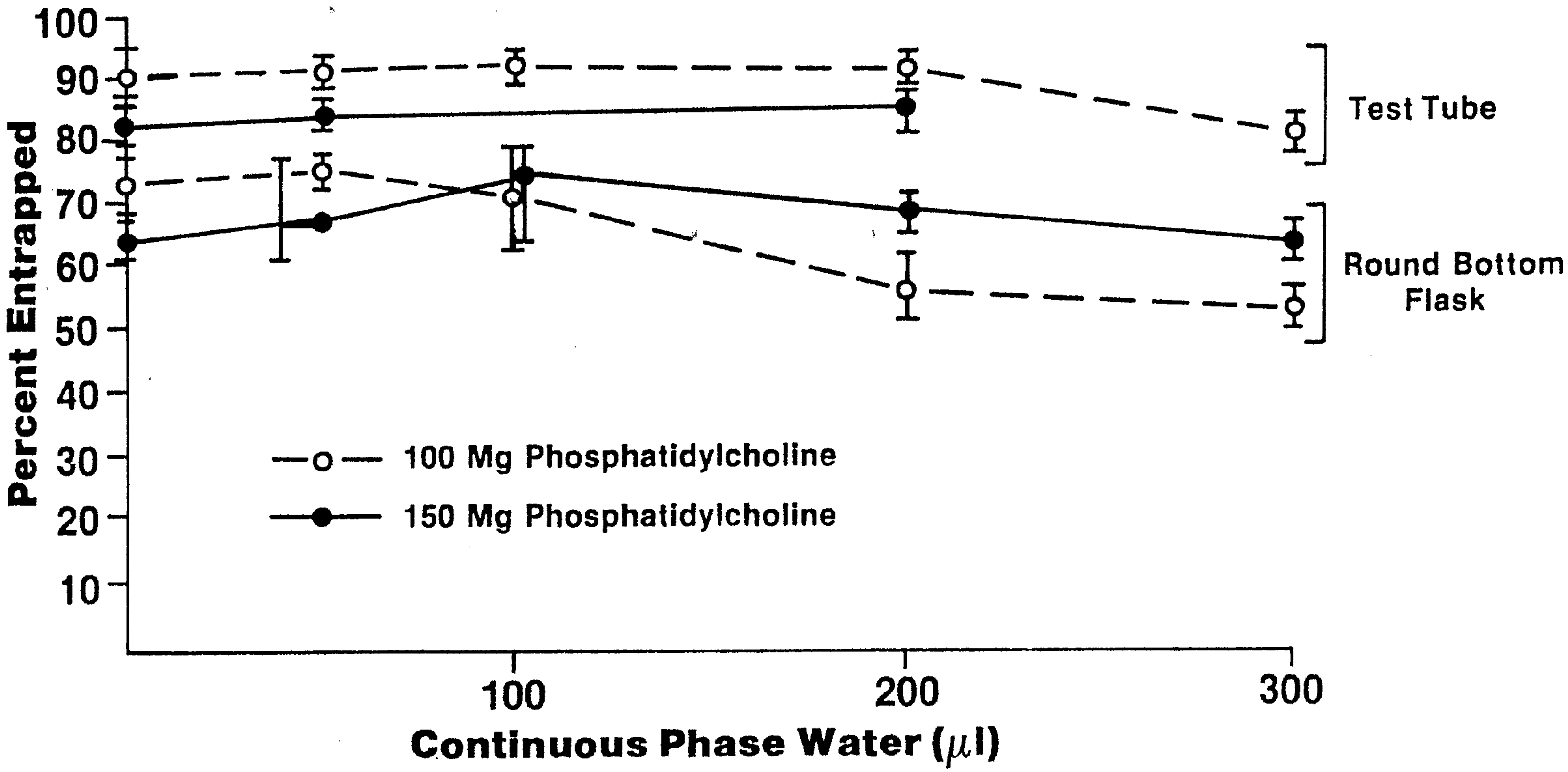

FIG.8

MLV-REV Prepared with PC/PA (9/1) - 2 Hour Exposure at 26°C

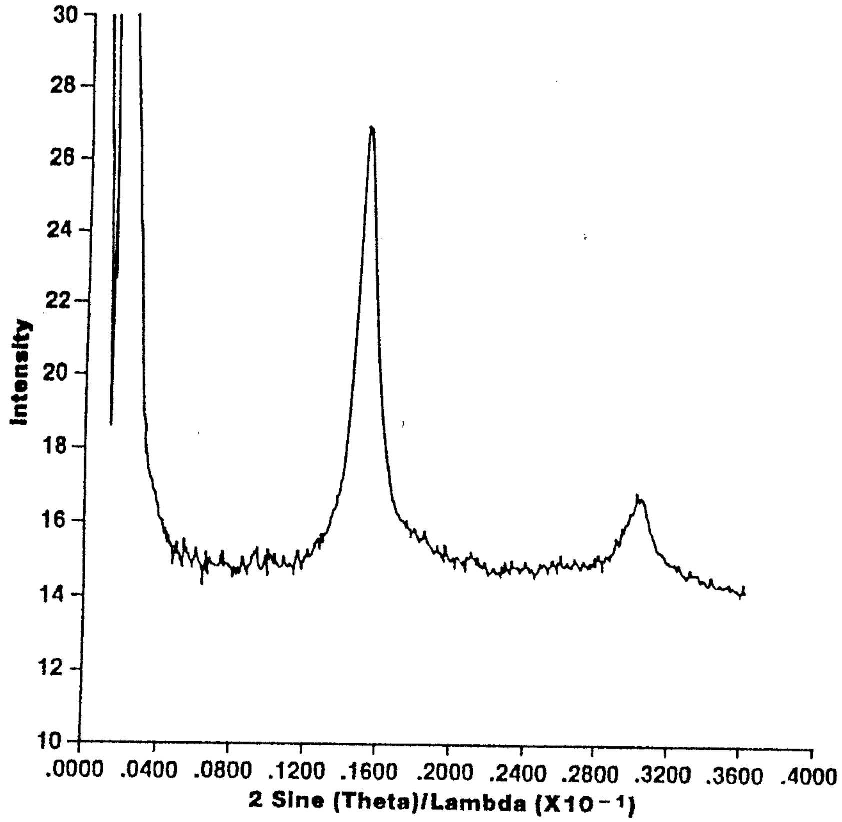


MLV-REV Prepared with PC/PS (9/1) - 2 Hour Exposure at 25.5°C

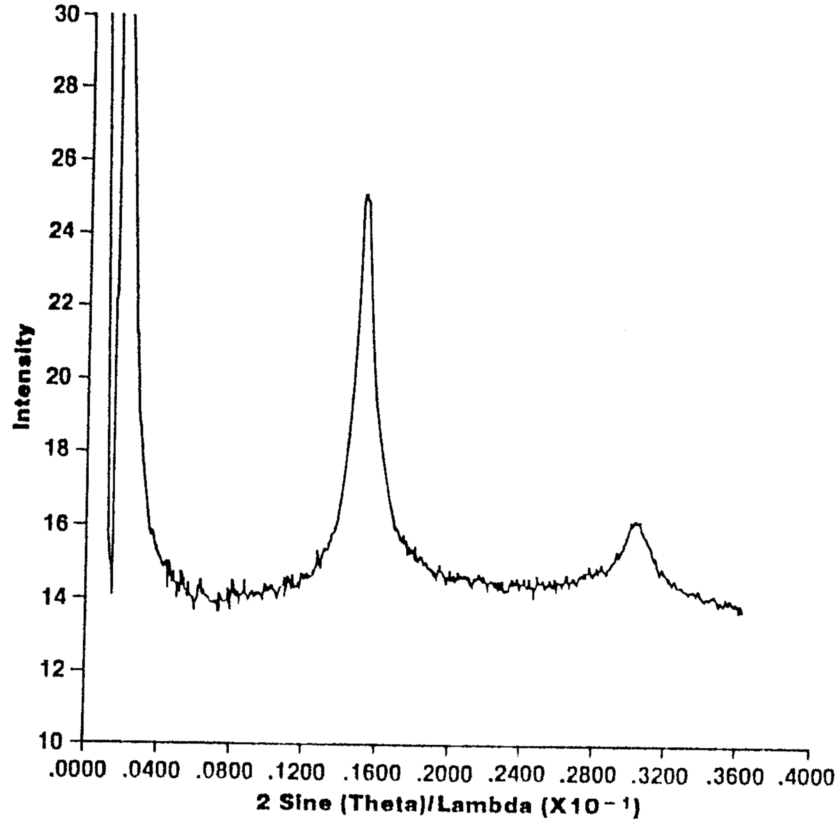

FIG. 9

**SPLV Prepared with PC/PS (9/1) - 15 Hour Exposure at 26°C**

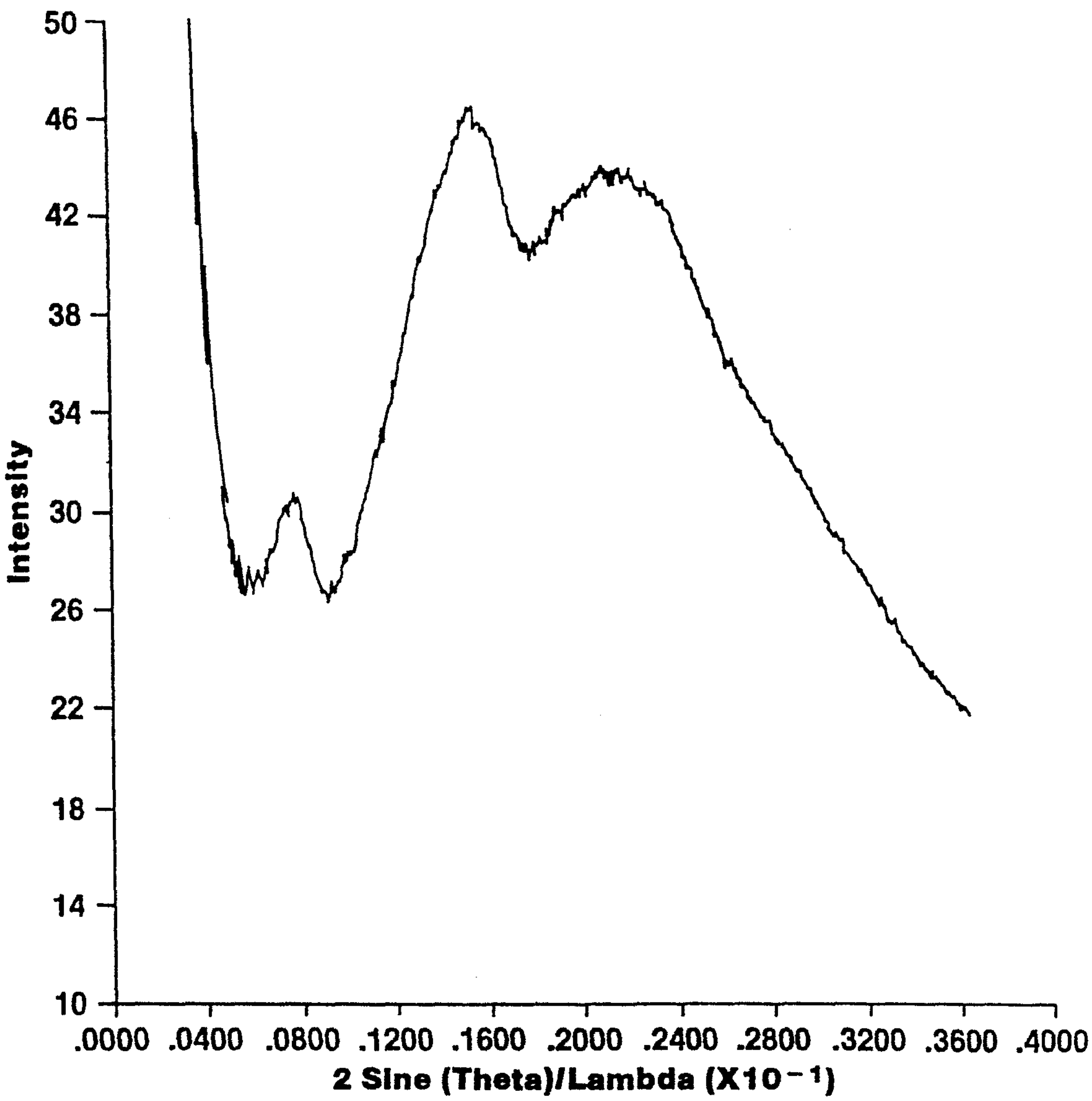

0179660

FIG. 10

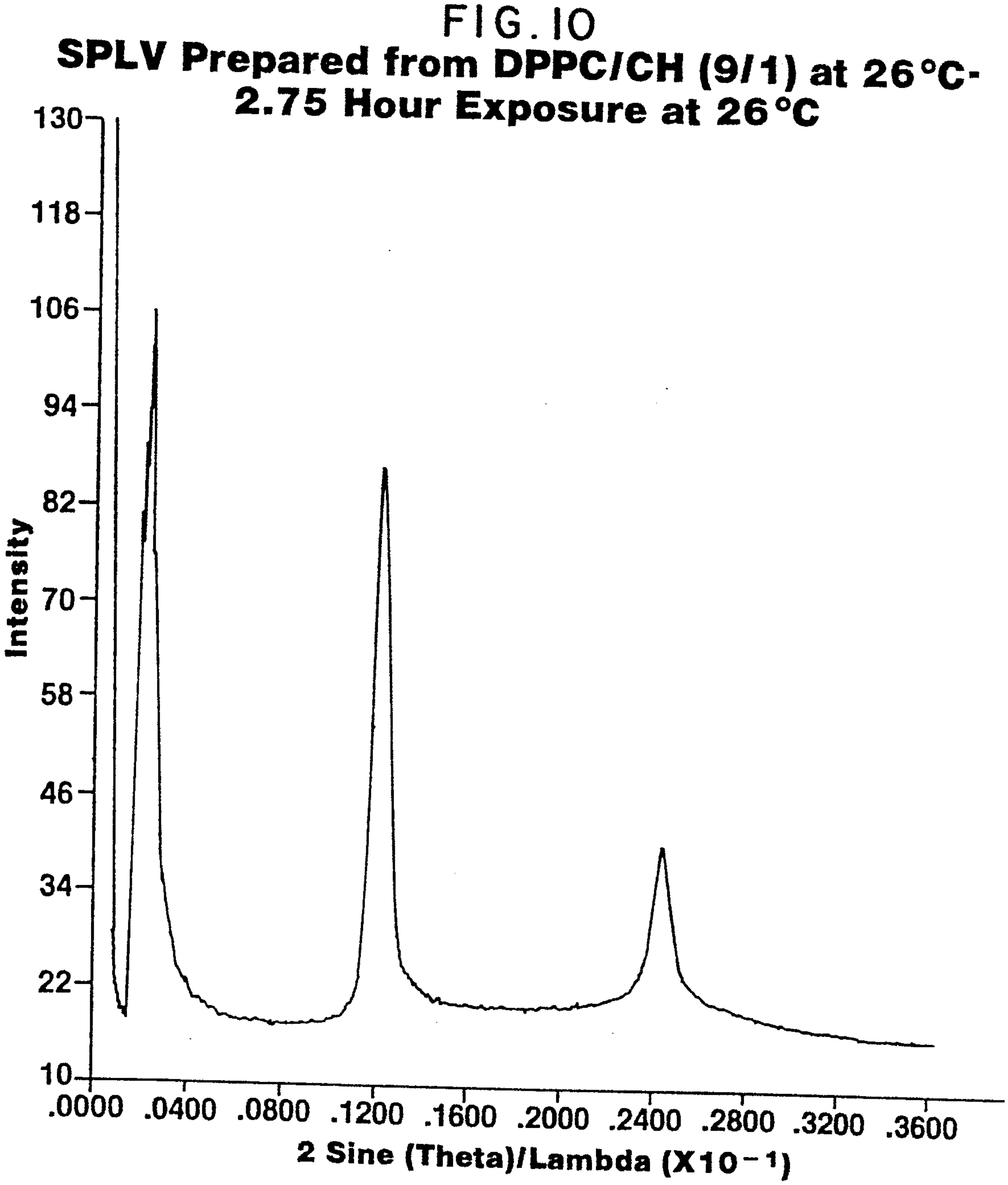

FIG.11

**SPLV Prepared with DPPC/CH (9/1) While Heating at 50°C During Vesicle Formation - 8 Hour Exposure at 26°C**

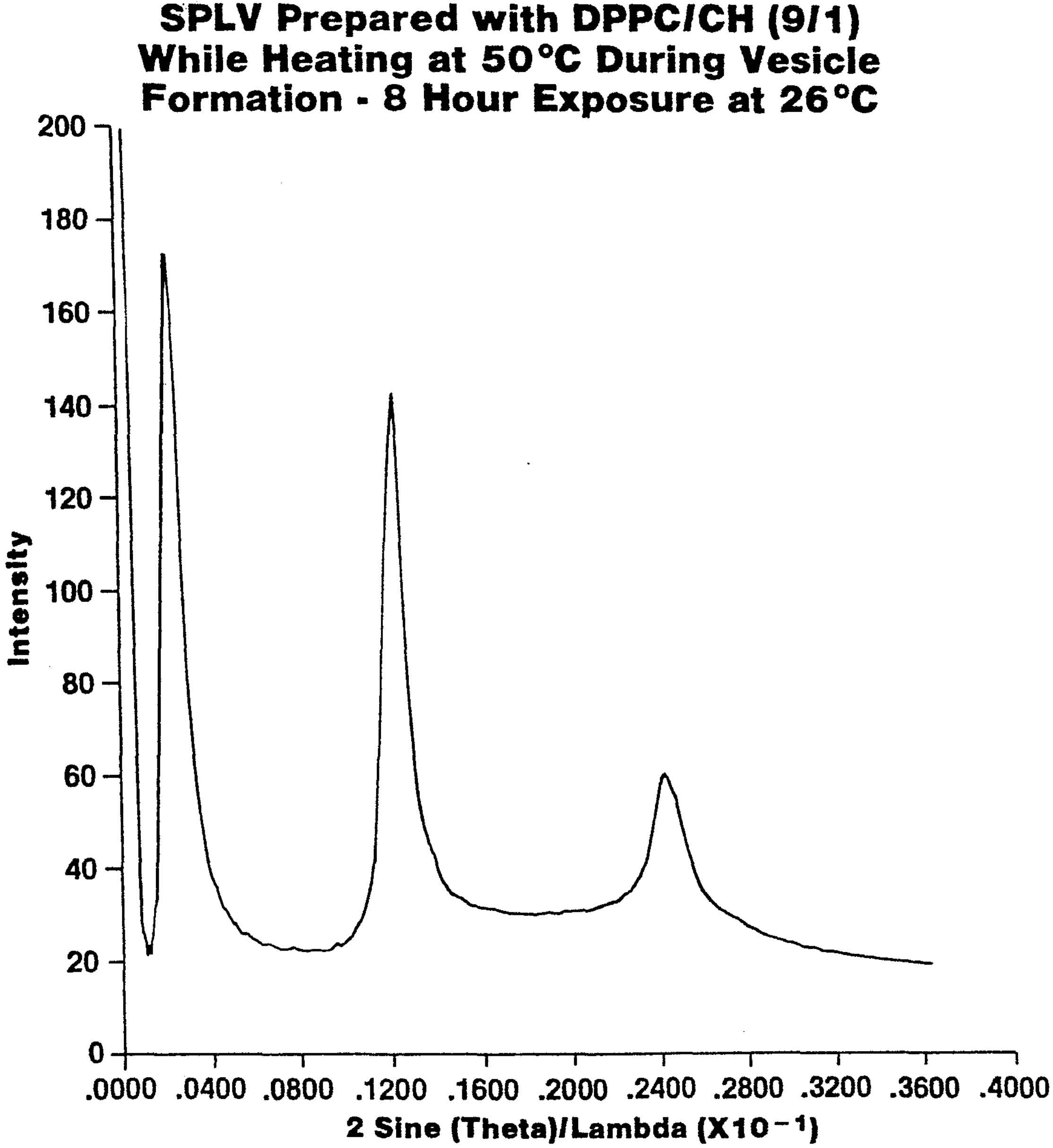

FIG.12

**MLV-REV Prepared with DPPC/CH (9/1) at 26°C - 3 Hour Exposure at 26°C**

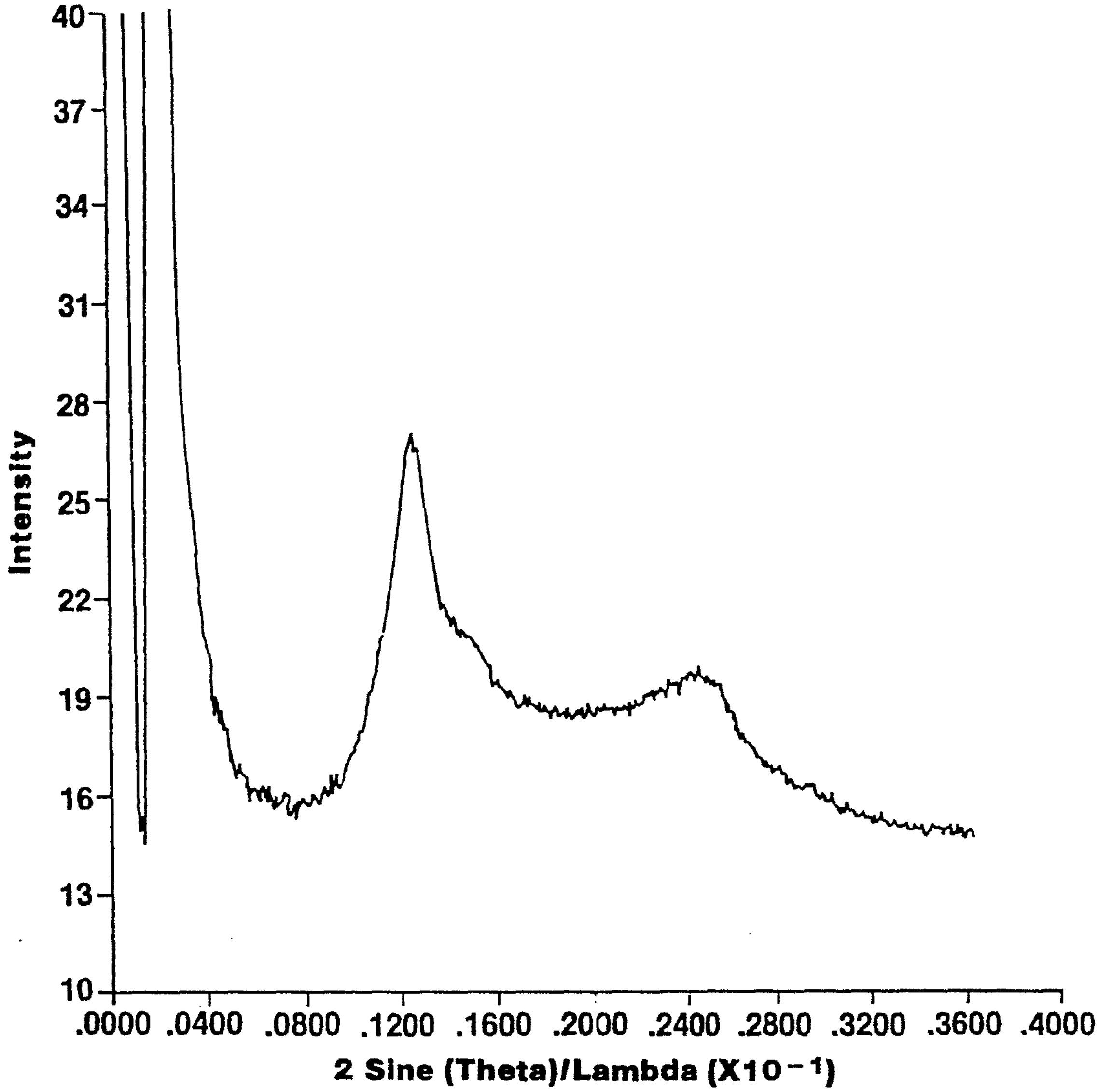

FIG.13

**MLV-REV Prepared with DPPC/CH (9/1) While Heating at 50°C During Vesicle Formation - 4 Hour Exposure at 26°C**

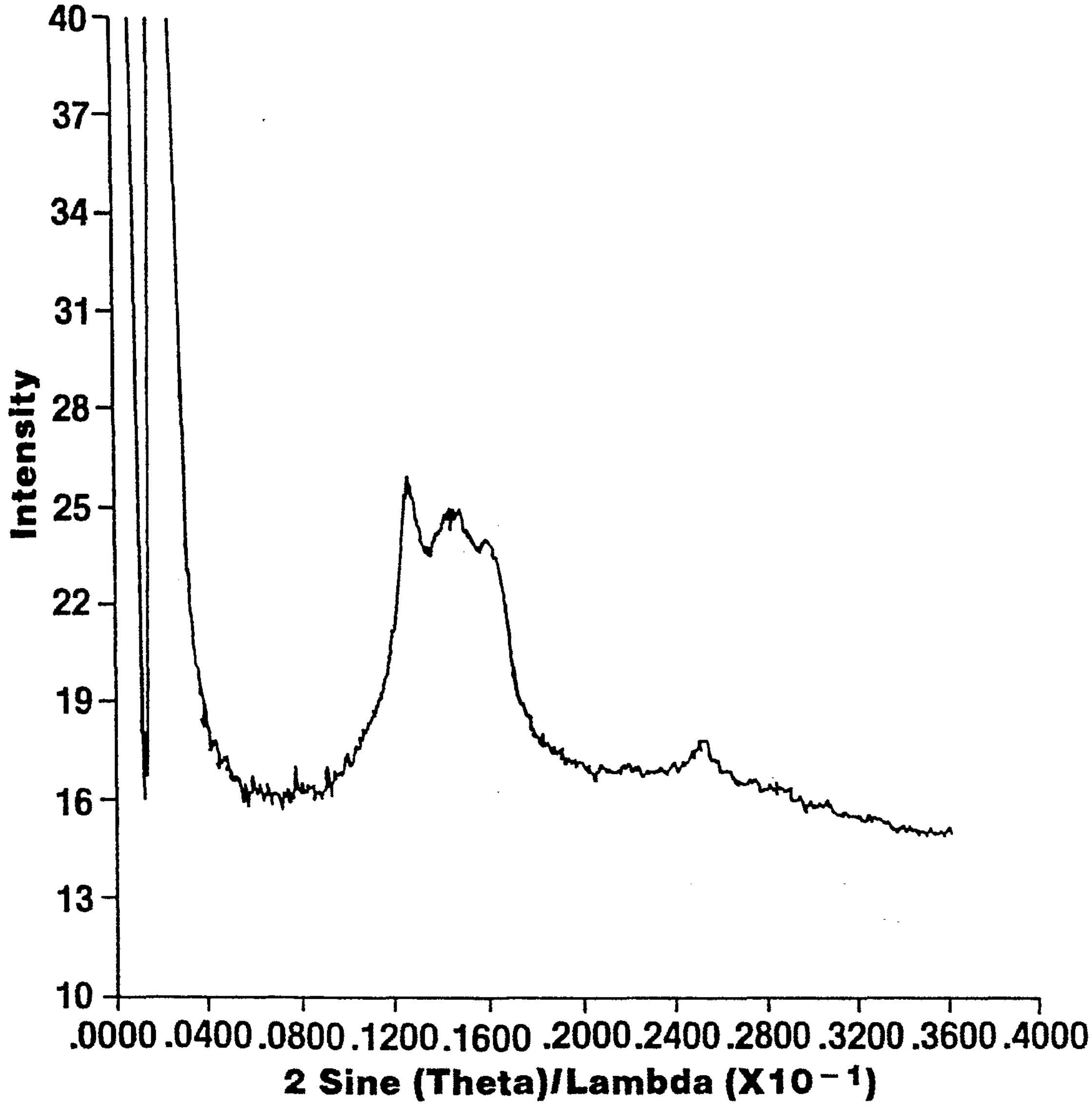

FIG. 14

**Temperature Dependent Long Spacing Neutral Vesicles**

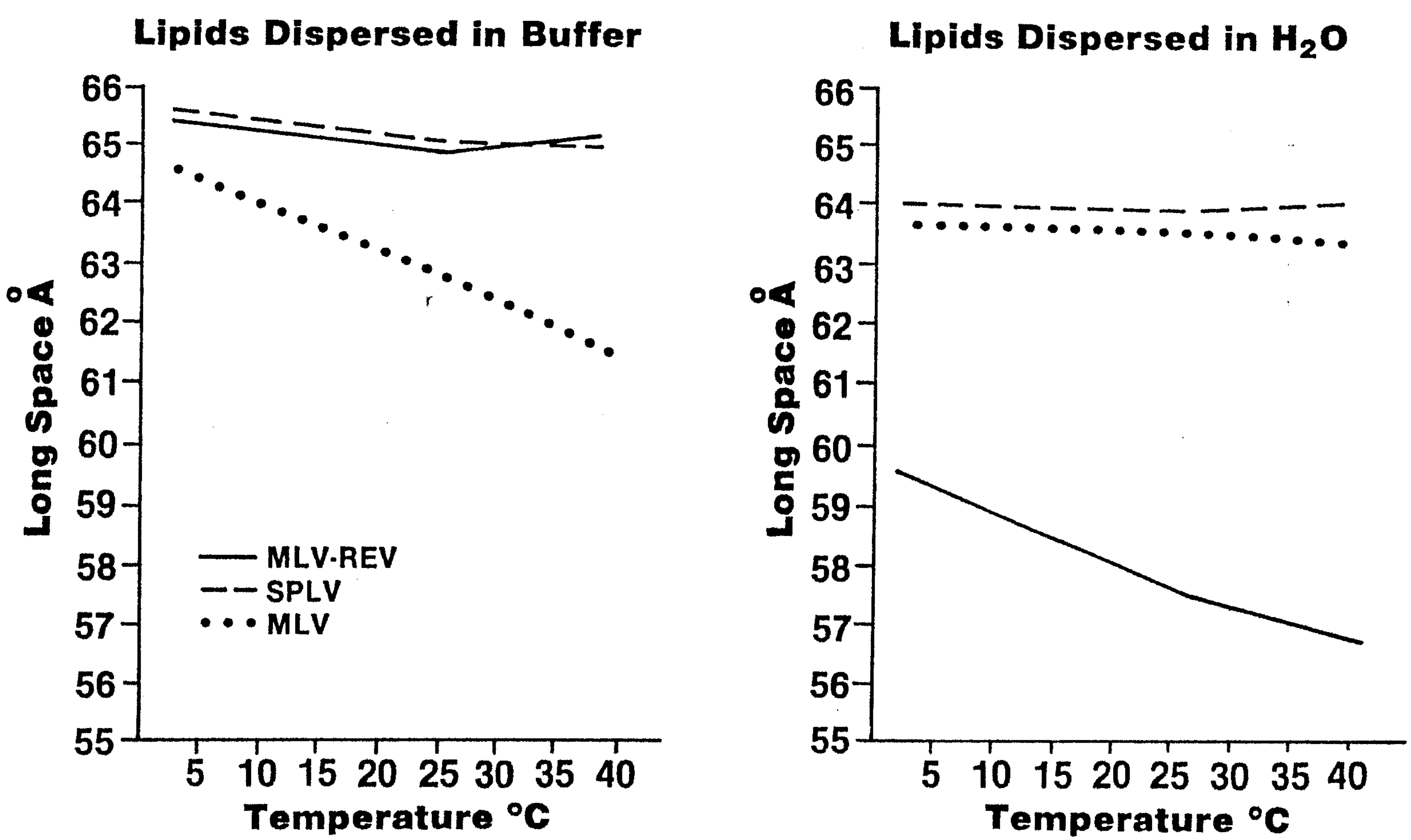

FIG. 15

**Leakage of 6-CF from Vesicles vs. Time**